# EUROPEAN PATENT SPECIFICATION

(11) **EP 1 694 305 B1**
(45) Date of publication and mention of the grant of the patent: **16.10.2019**
(21) Application number: 04801160.5
(22) Date of filing: 01.12.2004
(51) Int. Cl.: A61K 9/16, A61K 31/4418, A61K 9/20

(54) **PHARMACEUTICAL COMPOSITIONS COMPRISING LERCANIDIPINE**
PHARMAZEUTISCHE ZUSAMMENSETZUNGEN MIT LERCANIDIPIN
COMPOSITIONS PHARMACEUTIQUES CONTENANT DE LA LERCANIPIDINE

(30) Priority: 18.02.2004 DK 200400249; 01.12.2003 DK 200301778; 16.03.2004 US 55378704 P
(43) Date of publication of application: 30.08.2006
(73) Proprietor: Recordati Ireland Limited, Ringaskiddy County Cork (IE)
(72) Inventor: HOLM, Per, 2720 Vanløse (DK); NORLING, Tomas, 2800 Lyngby (DK)
(74) Representative: Serjeants LLP
(86) International application number: PCT/DK2004/000836
(87) International publication number: WO 2005/053689

(56) References cited:
- WO-A-03/014084
- US-A1- 2003 180 355

## Description

The present invention relates to compositions, particularly controlled release pharmaceutical compositions that achieve slow release of lercanidipine over an extended period of time, sufficient bioavailability to enable a once daily dosing after oral administration, significantly reduced food effect and increased bioavailability compared to commercially available lercanidipine containing products. Furthermore, compositions according to the invention are expected to reduce peak related side effects.

In particular the invention relates to solid pharmaceutical compositions comprising lercanidipine dissolved in a solid carrier formulated for oral administration and controlled release of lercanidipine.

### Background of the invention

Lercanidipine is a dihydropyridine calcium antagonist. As other calcium channel antagonists, it lowers blood pressure by relaxing arteriolar smooth muscle, which decreases peripheral vascular resistance. Lercanidipine produces no negative cardiac inotropism and only mild reflex tachycardia. It has a high affinity for and competitively antagonizes the dihyropyridine subunit of the L-type calcium channel.

Calcium channel antagonists are regarded as safe and have proven to be effective in all types of hypertension. Lercanidipine is a new type of calcium antagonist of the dihydropyridine class, which has demonstrated a powerful hypotensive effect and long duration of action in preclinical studies. Lercanidipine has been well tolerated in doses up to 30 mg and decreases blood pressure in a dose-dependent manner.

Calcium antagonists are contemplated to be renal protector due to their antihypertensive action. Such potential has been demonstrated in renal insufficiency and toxicity caused by cancer chemotherapy, radiocontrast agents, cyclosporine or aminoglycoside antibiotics. Calcium antagonists may also have protective effect on donor kidneys in kidney transplantation.

Lercanidipine (methyl 1,1-N-trimethyl-N-(3,3-diphenylpropyl) aminoethyl 1,4dihydro 6-dimethyl (3-nitrophenyl)pyridine-3,5-dicarboxylate) is a highly lipophilic dihydropyridine calcium antagonist with long duration of action and high vascular selectivity. The structural formula is 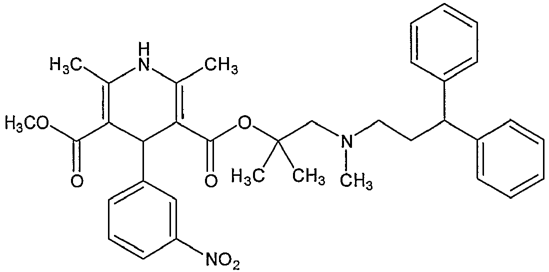 and the molecular weight is about 512.

Lercanidipine is normally used in a dose of 10 mg to 20 mg once daily, the maximum dose being about 30 mg daily. Lercanidipine is used for treating mild to moderate hypertension and is also expected to be useful in angina pectoris. It has also been beneficial in elderly patients with isolated systolic hypertension. The recommended starting dose of lercanidipine is given by mouth 10 mg once daily before food increased, if necessary, after at least 2 weeks to 20 mg daily. Lercanidipine is rapidly absorbed following oral administration and peak plasma levels occurring 1.5-3 hours following dosing, but it undergoes extensive saturable first-pass metabolism. The absorption is highly dependent on food intake, i.e. simultaneous intake of food increases the amount absorbed markedly (3-4 times). Lercanidipine is rapidly and widely distributed. It is more than 98% bound to plasma proteins. Lercanidipine is extensively metabolized to inactive metabolites and about 50% of the oral dose is excreted in urine. The terminal elimination t_{½} is about 2-5 hours. Lercanidipine is a substrate for CYP3A4 and is metabolized in the liver via CYP3A4 to a number of inactive metabolites that is eliminated via the kidneys. Elimination is essentially via the hepatic route.

By virtue of its high lipophilicity and high membrane coefficient, lercanidipine is said to combine a short plasma half-life with a long duration of action. Thus, the distribution of the drug into membranes of smooth muscle cells results in membrane controlled pharmacokinetics characterized by a prolonged pharmacological effect. In comparison to other calcium antagonists, lercanidipine is characterized by gradual onset and longer-lasting duration of action despite decreasing plasma levels.

As mentioned above, lercanidipine is administered orally and is therefore absorbed from the gastrointestinal tract. It has been observed that the absorption is influenced by the simultaneous ingestion of food. Thus, the extent of lercanidipine absorption (AUC) was greatest when it was taken orally together with a meal. This observation indicates that absorption is not complete after the recommended dose regimen, in which lercanidipine is taken without food. Accordingly, there is a need for increasing the bioavailability of oral dosage forms containing lercanidipine, so as the absorption in fasted state is equal or close to the absorption observed in the fed state. This type of formulation, together with a once daily administration (a controlled release formulation) would be highly appreciated, as it would improve the patient compliance significantly.

In general, it is known that the absorption and bioavailability of a therapeutically active substance can be affected by a variety of factors when administered orally. Such factors include taking the medication together with food in which case the absorption can either be reduced or enlarged. In the case of Lercanidipine the amount absorped, when taken together with a meal, is 3 to 4 times larger than if taken without food. This makes absorption irregular. The recommended dosing regime state that the product must be taken prior to a meal, the cause of which could be to avoid high peak levels with accompanying hypotension and/or headache. If this food effect is reduced or negated, the dose might be lowered and the plasma levels more reproducible. It is contemplated that a once daily formulation of lercanidipine, according to the invention might be taken any hours of the day, independently of meals and will secure 24 hours, reproducible therapeutic plasma level of Lercanidipine.

Lercanidipine is a substrate for cytochrome P450 IIIA4 (CYP3A4) isoenzyme. Many drug substances are substrates for P450 IIIA4 (CYP3A4) isoenzyme and P-glycoprotein and are extensively metabolized by the CYP3A4 isoenzyme in the gut wall and liver. Therefore, absorption and the subsequent elimination of systemically absorbed drug substances that are such substrates (e.g. lercanidipine etc.) may be influenced by other drug substances that affect this isoenzyme. Inhibitors of CYP3A4 may decrease the metabolism of e.g. lercanidipine and increase the drug levels, while inducers of CYP3A4 may increase the metabolism and decrease drug levels. Accordingly, drug substances like e.g. lercanidipine may be administered together with one or more CYP3A4 inhibitors like e.g. grapefruit juice in order to improve the overall bioavailability.

For oral administration, lercanidipine is currently formulated and marketed as tablets containing 10 mg under the trademark Zanidip® in some European countries. Lercanidipine is commercially available from Recordati S.p.A. (Milan, Italy) and can be prepared as described in EP 153016 and US 4705797 (both to Recordati S.p.A.).

US-A1-2003/0180355 discloses a method for treating hypertension in patients involving administration of the active substances lercanidipine and enalapril as well as a combination formulation comprising lercanidipine and enalapril.

There remains a need for new pharmaceutical compositions comprising lercanidipine and releasing lercanidipine in a controlled manner so as to prolong the therapeutic effect after a single dose to obtain a better therapeutic effect during day and night. Furthermore, there is a need for new lercanidipine compositions exhibiting sufficient or increased bioavailability of the active compound and/or reduced or eliminated food effect. In particular it is desired to obtain a larger uptake of the active compound, and this may in turn provide for a reduction of the administered dose and/or dosages, which in turn lead to a better patient compliance Since lercanidipine has been shown to exhibit adverse side effects the latter is also an important objective. Further, pharmaceutical compositions comprising lercanidipine and exhibiting a higher bioavailability of this compound may allow a reduction in the dose or dosage units taken by a patient, e. g. down to a single dose daily or less frequent, and may also reduce or negate the need for food to be takes at another point of time than with the dosage form thereby allowing patients more freedom on when the drug is taken.

Furthermore, it is contemplated that fluctuations in the plasma concentration versus time profile may be reduced due to a marked reduction in peak plasma concentration while the plasma concentration is maintained at a therapeutic level for an extended period of time.

### Summary of the invention

The inventors have found formulations and pharmaceutical compositions comprising lercanidipine or an analog or pharmaceutical acceptable salt thereof, which surprisingly show an increased bioavailability, especially controlled release formulations.

Accordingly, in a first aspect the present invention relates to a pharmaceutical composition comprising lercanidipine or an analog or a pharmaceutical acceptable salt thereof as an active substance and a pharmaceutically acceptable vehicle that is glyceryl monolaurate, glyceryl monocaprylate or glyceryl (mono)caprate, which composition upon oral administration to a mammal in need thereof releases the active substance in a controlled manner. Lercanidipine may be fully dissolved in the vehicle to form a solid solution at ambient temperature or may partly dissolved in the vehicle to form a mixture of solid dispersion and solid solution at ambient temperature or may be dispersed or suspended in the vehicle to form a liquid suspension or solid dispersion at ambient temperature.

In a second aspect, the invention relates to solid dosage forms comprising the pharmaceutical lercanidipine composition and one or more pharmaceutically acceptable excipients and optionally pharmaceutically acceptable additives. The solid dosage form of the invention provides an AUC value relative to that of commercially available Zanidips tablets of at least about 1.1, or at least about 1.2, or at least about 1.3, or at least about 1.4, or at least about 1.5, or at least about 1.75 or more, or at least about 2.0, or at least about 2.5, or at least about 3.0, the AUC values being determined under similar conditions; and provides a Cmax value relative to that of commercially available Zanidip tablets of at least about 1.1, or at least about 1.2, or at least about 1.3, or at least about 1.4, or at least about 1.5, or at least about 1.6 or more, or at least about 2.0, or at least about 2.5, or at least about 3.0, the Cmax values being determined under similar conditions.

Thus, the present invention fulfills the unmet need for pharmaceutical lercanidipine containing compositions notably for oral use that lead to an improved treatment of conditions with lercanidipine. The controlled release formulation of the invention shows improved bioavailability which results in improved treatment because it will be possible
to obtain the same therapeutic response with a single dosing of lercanidipine once daily, possibly with a lower daily dosing (compared to commercially available Zanidip® tablets). This in turn may lead to a reduction in dose-related side effects. Furthermore, it is contemplated that the controlled release formulation if the invention reduces the peak values on the plasma curves and secure 24 hours trough level above the therapeutic plasma concentration.

### Description of the invention

### Definitions

As used herein, the term "active substance", "active ingredient" or "active pharmaceutical ingredient" means any component that is intended to furnish pharmacological activity or other direct effect in the diagnosis, cure, mitigation, treatment, or prevention of disease, or to affect the structure or any function of the body of man or other animals. The term includes those components that may undergo chemical change in the manufacture of the drug product and are present in the drug product in a modified form intended to furnish the specified activity or effect.

In the present context, the term "lercanidipine" includes any stereoisomer, enantiomer, isomer form thereof as well as lercanidipine in any crystal, amorphous or polymorphous form. A composition of the invention may also include one or more further
therapeutically, prophylactically and/or diagnostically active substances.

In the present context, the term "hydrophilic" describes that something 'likes water', i.e. a hydrophilic molecule or portion of a molecule is one that typically is electrically polarized and capable of forming hydrogen bonds with water molecules, enabling it dissolve more readily in water than in oil or other "non-polar" solvents.

In the present context, the term "amphiphilic" describes a molecule (as a surfactant) having a polar water-soluble group attached to a water-insoluble hydrocarbon chain. Thus, one end of the molecule is hydrophilic (polar) and the other is hydrophobic (non-polar).

In the present context, the term "hydrophobic" denotes a compound tending to be electrically neutral and non-polar, and thus preferring other neutral and nonpolar solvents or molecular environments.

As used herein, the term "water-miscible" denotes a compound being fully or partly miscible with water. For example, certain polar lipids are partly water-miscible.

As used herein, the term "vehicle" means any solvent or carrier in a pharmaceutical product that has no pharmacological role. For example, water is the vehicle for xilocaine and propylene glycol is the vehicle for many antibiotics.

In the present context, the term "solid dispersion" denotes a drug or active ingredient or substance dispersed on a particulate level in an inert vehicle, carrier, diluent or matrix in the solid state, i.e. usually a fine particulate dispersion.

In the present context, the term "solid solution" denotes a drug or active ingredient or substance dissolved on a molecular level in an inert vehicle, carrier, diluent or matrix in the solid state.

The term "drug" means a compound intended for use in diagnosis, cure, mitigation, treatment, or prevention of disease in man or other animals.

In this context, the term "dosage form" means the form in which the drug is delivered to the patient. This could be parenteral, topical, tablet, oral (liquid or dissolved powder), suppository, inhalation, transdermal, etc.

As used herein, the term "bioavailability" denotes the degree means to which a drug or other substance becomes available to the target tissue after administration.

As used herein, the term "bioequivalency" denotes a scientific basis on which generic and brand name drugs are compared with one another. For example, drugs are bioequivalent if they enter circulation at the same rate when given in similar doses under similar conditions. Parameters often used in bioequivalence studies are tₘₐₓ, cₘₐₓ, AUC_{0-infinity}, AUC₀₋ₜ. Other relevant parameters may be W₅₀, W₇₅ and/or MRT. Accordingly, at least one of these parameters may be applied when determining whether bioequivalence is present. Furthermore, in the present context, two compositions are regarded as bioequivalent if the value of the parameter used is within 80-125% of that of Zanidip® or a similar commercially available lercanidipine-containing product used in the test.

In the present context "tₘₐₓ" denotes the time to reach the maximal plasma concentration (cₘₐₓ) after administration; AUC_{0-infinity} or AUC denotes the area under the plasma concentration versus time curve from time 0 to infinity; AUC₀₋ₜ denotes the area under the plasma concentration versus time curve from time 0 to time t; W₅₀ denotes the time where the plasma concentration is 50% or more of Cₘₐₓ; W₇₅ denotes the time where the plasma concentration is 75% or more of Cₘₐₓ; and MRT denotes mean residence time for lercanidipine (and/or an analog thereof).

In this context, the term "medicine" means a compound used to treat disease, injury or pain. Medicine is designated "prophylactic, " i. e. the art of preserving health, and "therapeutic", i. e. the art of restoring health.

In the present context, the terms "controlled release" and "modified release" are intended to be equivalent terms covering any type of release of lercanidipine from a composition of the invention that is appropriate to obtain a specific therapeutic or prophylactic response after administration to a subject. A person skilled in the art knows how controlled release/modified release differs from the release of plain tablets or capsules. The terms "release in a controlled manner" or "release in a modified manner" have the same meaning as stated above. The terms include slow release (that results in a lower Cₘₐₓ and later tₘₐₓ, but the half-life is unchanged), extended release (that results in a lower Cₘₐₓ, later tₘₐₓ, but apparent the half-life is longer) ; delayed release (that result in an unchanged Cₘₐₓ, but lag time and, accordingly, tₘₐₓ is delayed, and the half-life is unchanged) as well as pulsatile release, burst release, sustained release, prolonged release, chrono-optimized release, fast release (to obtain an enhanced onset of action) etc. Included in the terms is also e. g. utilization of specific conditions within the body e. g. different enzymes or pH changes in order to control the release of the drug substance.

In this context, the term "erosion" or "eroding" means a gradual breakdown of the surface of a material or structure, for example of a tablet or the coating of a tablet.

The present invention provides pharmaceutical compositions and solid dosage forms for improved treatment of conditions that respond to lercanidipine therapy.

The pharmaceutical compositions and solid dosage forms of the invention comprise lercanidipine or a pharmaceutical acceptable salt thereof and a pharmaceutically acceptable vehicle. Lercanidipine is fully dissolved in the vehicle to form a solid solution at ambient temperature.

The vehicle of the present invention is glyceryl monolaurate, glyceryl monocaprylate or glyceryl (mono)caprate.

The pharmaceutical composition of the invention may be in the form of particles, i. e. in particulate form.

The concentration of lercanidipine in the vehicle is preferably less than about 30 w/w%, based on the total weight of the active substance and the vehicle; or is at least about 1 w/w%, based on the total weight of the active substance and the vehicle.

Upon oral administration to a mammal in need thereof, the pharmaceutical composition or the solid dosage form of the invention may exhibit an AUC/AUC_{control} value of at least about 1.1, the AUC values being determined under similar conditions. The composition used as a control is given in the same dosage and is a commercially available lercanidipine composition intended for oral administration. In the present context, the control composition is Zanidip® tablets.

As it appears from the examples herein the bioavailability obtained after administration of a composition according to the invention is markedly improved. Thus, in specific embodiments, the AUC/AUC_{control} value is at least about 1.2, such as, e. g., about 1.3
or more, about 1.5 or more, about 1.75 or more, about 1.8 or more, about 1.9 or more, about 2.0 or more, about 2.5 or more, about 2.75 or more, about 3.0 or more, about 3.25 or more, about 3.5 or more, about 3.75 or more, about 4.0 or more, about 4.25 or more, about 4.5 or more, about 4.75 or more, or about 5 or more, the AUC values being determined under similar conditions.

After oral administration of a pharmaceutical composition according to the present invention it is contemplated that the plasma concentration versus time profile show an extended period of time in which the plasma concentration is maintained within the therapeutic window (i.e. the plasma concentration leads to a therapeutic effect) without leading to side effects. Thus, a reduction in peak concentration may also be observed. In a specific embodiment, it may be of interest to provide a pharmaceutical composition (in particulate or solid dosage form, e.g. tablet form) comprising lercanidipine together with one or more pharmaceutically acceptable excipient, wherein the composition upon oral administration to a mammal in need thereof release lercanidipine or a derivative or analogue thereof in a controlled manner and exhibits a Cₘₐₓ that is at the most about 95% of that of Cₘₐₓ for Zanidip® tablets such as, e.g., at the most about 90%, at the most about 85%, at the most about 80%, at the most about 75%, at the most about 70%, at the most about 65%, at the most about 60%, at the most about 55%, at the most about 50%, at the most about 45% or at the most about 40%.

However, a reduction in peak concentration should not lead to a decrease in therapeutic effect. Accordingly, the present invention also relates to a pharmaceutical composition, wherein W₅₀ is at least about 2 hours, such as, e.g., at least about 3 hours, at least about 4 hours, at least about 5 hours, at least about 6 hours, at least about 7 hours, at least about 8 hours, at least about 9 hours, at least about 10 hours, at least about 12 hours, at least about 14 hours, at least about 16 hours, at least about 18 hours or at least about 20 hours. Furthermore or moreover, in one embodiment, a composition according to the invention has a C_{diff}= [Cₘₐₓ - Cₜ (t is at least 6 hours and at the most 16 hours, normally t is set to 12 hours)] that is less than that of Zanidip® tablets under the same conditions. If C_{diff} for Zanidip® tablets is set to 100 then C_{diff} of a composition according to the invention may be 90 or less such as, e.g., about 85 or less, about 80 or less, about 75 or less, about 70 or less, about 65 or less, about 60 or less, about 55 or less, about 50 or less, about 45 or less or about 40 or less.

Thus, it has been found that the pharmaceutical compositions according to the invention exhibit surprisingly higher bioavailability compared to commercially available formulations such as Zanidip®. In fact the bioavailability of lercanidipine can according to the invention be increased by over 3-4 times compared with the said commercially available product. Accordingly, the current daily frequency of dosing may be reduced by administration of a composition of the invention. It is contemplated that the current daily dosing once or twice daily can be reduced to a once daily or even once every second day dosing.

A pharmaceutical composition according to the invention releases lercanidipine in a controlled manner in order to extend the therapeutic action of lercanidipine. In a particularly interesting aspect, the composition is in the form of a tablet. The release may suitably be pH independent, e.g. by providing the composition with a controlled release coating such as, e.g. a cellulose based coating like e.g. ethylcellulose, or by use of a controlled release matrix. A combination may of course also be employed.

In general, the change in bioavailability and/or the changes in other bioavailability related parameters are normally determined by *in vivo* studies in a suitable animal model testing the compositions in question together with e.g. Zanidip® or a similar commercially available lercanidipine-containing product. The use of a dog model for establishing evidence of the bioavailability of certain formulations is general practice in the pharmaceutical industry.

The studies relevant for lercanidipine are non-randomized, cross-over studies, where each dog is its own control. Four dogs and four treatments are normally applied. As no i.v. injections are given, the bioavailabilities obtained are relative.

Further it has surprisingly been found that simultaneous food intake in order to obtain a maximal uptake of lercanidipine is not necessary, thus being significantly reduced or even completely abolished.

Thus, the pharmaceutical compositions according to the invention provide significant higher bioavailability of lercanidipine, which may reduce the daily intake of lercanidipine, reduce the administration frequency, improve the therapeutic efficacy and reduce or abolish the influence of food on the absorption, which provide for a higher degree of freedom for the recipient of the pharmaceutical compositions, and consequently the patients acceptance and/or compliance may be significantly improved. Furthermore, the compositions provide a reduction in side effects, especially side effect related to a high peak concentration and provide for an extended release of lercanidipine leading to a better therapy such as, e.g., administration once daily with improved therapeutic efficacy.

As mentioned above, besides improving the overall bioavailability, one of the major challenges with respect to formulation of lercanidipine compositions is to avoid an adverse food effect. In general, lercanidipine is much better absorbed when it is administered orally together with food. A great variation in bioavailability is therefore seen following administration with or without food. This dependency makes it difficult to give precise guidelines as to how large a dose that should be administered and, furthermore, it requires information to the patient about the dosing regime. The present invention aims at providing compositions wherein the adverse food effect is reduced. Thus, the present invention provides a composition, which does not exhibit a significant adverse food effect after administration of the composition to a mammal in need of such a treatment as evidenced by a value of (AUC_{fed}/AUC_{fasted}) of at least about 0.85 with a lower 90% confidence limit of at least 0.75.

More specifically, a pharmaceutical composition according to the invention has a value of (AUC_{fed}/AUC_{fasted}) of at the most about 3, such as, e.g. at the most about 2.5, at the most about 2.0, at the most about 1.5, at the most about 1, such as, e.g., about 0.9 or more, about 0.95 or more, about 0.97 or more or about 1 or more such as, e.g., up to about 1.1 or up to about 1.2.

A further advantage of a composition of the present invention is the possibility of obtaining an effective therapeutic response with a decreased dosage and/or a decreased administration frequency compared to traditional oral treatment. Accordingly, upon oral administration to a mammal in need thereof a pharmaceutical composition according to the invention releases lercanidipine in a controlled manner and the composition is essentially bioequivalent with Zanidip® or a similar commercially available lercanidipine-containing product when administered in a dose that is at the about most about 85% w/w such as, e.g., at the most about 80% w/w, at the most about 75%, at the most about 70% w/w, at the most about 65% w/w, at the most about 60% w/w, at the most about 55% w/w or at the most about 50% w/w of the dose of lercanidipine administered in the form of Zanidip® or a similar commercially available lercanidipine-containing product.

In some embodiments of the invention, the compositions are designed to release lercanidipine in a pH-dependent manner so as to avoid release in the stomach and delay the release until the composition after oral administration passes the stomach and reaches the small intestine. Delayed release is mainly brought about by some kind of enteric coating. Whereas semi-permeable coating will show some kind of delayed release, it does not significantly delay the release. Additionally it requires a certain amount of time to release the content. The coating may be a pH dependant coating. This type of coating is very resistant to release of drug until a certain pH is reached. When pH changes as little as between about 0.05 to about 0.4 upwards or downwards, the film alters properties and becomes permeable. Examples of pH-sensitive polymers, which are relatively insoluble and impermeable at the pH of the stomach, but which are more soluble and permeable at the pH of the small intestine and colon include, but not limited to:
Polyacrylamides, phthalate derivatives such as acid phthalates of carbohydrates, amylose acetate phthalate, cellulose acetate phthalate, other cellulose ester phthalates, cellulose ether phthalates, hydroxypropylcellulose phthalate, hydroxypropylethylcellulose phthalate, hydroxypropylmethylcellulose phthalate, methylcellulose phthalate, polyvinyl acetate phthalate, polyvinyl acetate hydrogen phthalate, sodium cellulose acetate phthalate, starch acid phthalate, styrene-maleic acid dibutyl phthalate copolymer, styrene-maleic acid polyvinylacetate phthalate copolymer, styrene and maleic acid copolymers, polyacrylic acid derivatives such as acrylic acid and acrylic ester copolymers, polymethacrylic acid and esters thereof, poly acrylic methacrylic acid copolymers, shellac, and vinyl acetate and crotonic acid copolymers.

pH-sensitive polymers of specific interest include shellac; phthalate derivatives, particularly cellulose acetate phthalate, polyvinylacetate phthalate, and hydroxypropylmethylcellulose phthalate; polyacrylic acid derivatives, particularly polymethyl methacrylate blended with acrylic acid and acrylic ester copolymers; and vinyl acetate and crotonic acid copolymers.

Increasing the bioavailability, the Area Under the Curve, will normally reduce the intra- and inter- variability related to absorption of a drug substance. This is particularly true; whenever the low and impaired bioavailability is a consequence of poor water solubility. It is contemplated that compositions according to the invention will provide CV's on Area under Curve data that are smaller than or equivalent with Zanidip® and like products.

Furthermore, it is envisaged that a pharmaceutical composition comprising lercanidipine together with one or more pharmaceutically acceptable excipient - and wherein the composition upon oral administration to a mammal in need thereof releases lercanidipine or an analogue thereof in a controlled manner (dependent on the design of the composition, this may be a pH-dependant or a pH-independent manner) - reduces inter- and/or intra-individual variations compared to those of Zanidip® administered under the same conditions and in a dose that provides an equivalent therapeutic effect.

In a specific aspect, the invention provides a pharmaceutical composition or a solid dosage form that releases lercanidipine in an extended manner so as to enable a long maintenance of the therapeutic effect. Accordingly, the invention relates to a pharmaceutical composition (e.g. in particulate or in a solid dosage unit form like e.g. tablets or capsules) form comprising lercanidipine together with one or more pharmaceutically acceptable excipient, wherein the composition upon oral administration to a mammal in need thereof in a controlled manner releases at least about 50% w/w of the total amount of lercanidipine and/or an analogue thereof within about 15 hours such as, e.g., within about 12 hours.

In specific embodiments of the invention, a composition releases lercanidipine according to in one or more of the following requirements. The release may be in vivo in the gastrointestinal tract and/or *in vitro* as tested by a suitable *in vitro* dissolution test e.g. according to the European Pharmacopoeia (Ph.Eur.) or, preferably, the US Pharmacopoeia (USP):
i) At least about 50% w/w lercanidipine is released after at least about 2 hours, such as, e.g., at least about 3 hours, at least about 4 hours, at least about 5 hours, at least about 6 hours, at least about 7 hours, at least about 8 hours, at least about 9 hours, at least about 10 hours, at least about 11 hours, at least about 12 hours, at least about 13 hours, at least about 14 hours, at least about 15 hours, at least about 16 hours, or at least about 17 hours, and/or
ii) at least about 60% w/w lercanidipine is released after at least about 2 hours, such as, e.g., at least about 3 hours, at least about 4 hours, at least about 5 hours, at least about 6 hours, at least about 7 hours, at least about 8 hours, at least about 9 hours, at least about 10 hours, at least about 11 hours, at least about 12 hours, at least about 13 hours, at least about 14 hours, at least about 15 hours, at least about 16 hours, or at least about 17 hours, and/or
iii) at least about 70% w/w lercanidipine is released after at least about 3 hours, such as, e.g., at least about 4 hours, at least about 5 hours, at least about 6 hours, at least about 7 hours, at least about 8 hours, at least about 9 hours, at least about 10 hours, at least about 11 hours, at least about 12 hours, at least about 13 hours, at least about 14 hours, at least about 15 hours, at least about 16 hours, at least about 17 hours, or at least about 18 hours, and/or
iv) at least 80% w/w lercanidipine is released after at least 4 hours, such as, e.g., at least 5 hours, at least 6 hours, at least 7 hours, at least 8 hours, at least 9 hours, at least 10 hours, at least 11 hours, at least 12 hours, at least 13 hours, at least 14 hours, at least 15 hours, at least 16 hours, at least 17 hours, at least 18 hours, at least 19 hours or at least 20 hours and/or
v) at least 85% w/w such as, e.g., at least about 90% w/w or at least about 95% w/w lercanidipine is released after at least 6 hours, at least 7 hours, at least 8 hours, at least 9 hours, at least 10 hours, at least 11 hours, at least 12 hours, at least 13 hours, at least 14 hours, at least 15 hours, at least 16 hours, at least 17 hours, at least 18 hours, at least 19 hours, at least 20 hours, at least 21 hours, at least 22 hours, at least 23 hours, or at least 24 hours, and/or
vi) at the most about 20% w/w is released within about 10 hours such as, e.g., within about 9 hours, within about 8 hours, within about 7 hours, within about 6 hours, within about 5 hours, within about 4 hours, within about 3 hours or within about 2 hours, and/or
vii) at the most about 30% w/w is released within about 12 hours such as, e.g., within about 11 hours, within about 10 hours, within about 9 hours, within about 8 hours, within about 7 hours, within about 6 hours, within about 5 hours, within about 4 hours, or within about 3 hours, and/or
viii) at the most about 40% w/w is released within about 13 hours such as, e.g., within about 12 hours, within about 11 hours, within about 10 hours, within about 9 hours, within about 8 hours, within about 7 hours, within about 6 hours, within about 5 hours, or within about 4 hours, and/or
ix) at the most about 50% w/w is released within about 14 hours such as, e.g., within about 13 hours, within about 12 hours, within about 11 hours, within about 10 hours, within about 9 hours, within about 8 hours, within about 7 hours, within about 6 hours, within about 5 hours, or within about 4 hours, and/or
x) at the most 15% w/w is released within the first hour after administration or after start of the *in vitro* dissolution test, and/or
xi) at the most 20% w/w is released within 2 hours after administration or after start of the *in vitro* dissolution test, and/or
xii) at the most 25% w/w such as, e.g., from about 5% to about 25% w/w is released within 3 hours after administration or after start of the *in vitro* dissolution test, and/or
xiii) at the most 30% w/w such as, e.g., from about 10% to about 25% w/w is released within 4 hours after administration or after start of the *in vitro* dissolution test, and/or
xiv) at the most 45% w/w such as, e.g., from about 20% to about 45% w/w is released within 6 hours after administration or after start of the *in vitro* dissolution test, and/or
xv) at the most 55% w/w such as, e.g., from about 35% to about 55% w/w is released within 8 hours after administration or after start of the *in vitro* dissolution test, and/or
xvi) at the most 70% w/w such as, e.g., from about 35% to about 70% w/w is released within 10 hours after administration or after start of the *in vitro* dissolution test, and/or
xvii) at the most 80% w/w such as, e.g., from about 40% to about 80% w/w is released within 12 hours after administration or after start of the *in vitro* dissolution test, and/or

In other specific embodiments, upon oral administration to a mammal in need thereof a composition according to the invention releases at least about 50% w/w of the total amount of lercanidipine and/or an analogue thereof within about 10 hours such as, e.g., within about 8 hours, within about 6 hours, within about 4 hours or within about 3 hours.

In a further embodiment, upon oral administration to a mammal in need thereof a pharmaceutical composition according to the invention releases at least about 55% w/w such as, e.g., about 60% w/w or more, about 65% w/w or more, about 70% w/w or more, about 75% w/w or more or about 80% w/w or more of the total amount of lercanidipine and/or an analogue thereof within about 15 hours such as, e.g., within about 12 hours, within about 10 hours, within 8 hours or within about 6 hours.

Furthermore or alternatively, at least about 50% w/w of the total amount of lercanidipine and/or an analogue thereof is released within 15 hours such as, e.g., within about 12 hours, when tested in an in vitro dissolution test and employing a dissolution medium comprising a buffer having pH 7.5. Guidance for a suitable dissolution test is described in the Examples herein, but variations with respect to the specific method employed and the ingredients contained in the dissolution medium etc. are within the scope of the present invention. A person skilled in the art will know how to carry out a suitable dissolution test e.g. with guidance from USP, Ph.Eur. and the like. Suitable conditions for the *in vitro* dissolution test are employing USP dissolution test (paddle method) and a buffer pH 7.5 containing 0.75% sodium lauryl sulfate as dissolution medium.

In other embodiments, the following conditions are fulfilled with respect to *in vitro* dissolution test:
i) at least about 50% w/w of the total amount of lercanidipine or an analogue thereof is released within about 10 hours such as, e.g., within about 8 hours, within about 6 hours, within about 4 hours, within about 3 hours or within about 2 hours, when tested in an in vitro dissolution test and employing a dissolution medium comprising a buffer having pH 7.5
ii) at least about 50% w/w of the total amount of lercanidipine or an analogue thereof is released within about 1.5 hours such as, e.g., within about 1 hour, within about 0.75 hours, within about 0.5 hours or within about 20 minutes, when tested in an in vitro dissolution test and employing a dissolution medium comprising a buffer having pH 7.5.
iii) at least about 55% w/w such as, e.g., about 60% w/w or more, about 65% w/w or more, about 70% w/w or more, about 75% w/w or more or about 80% w/w or more of the total amount of lercanidipine or an analogue thereof is released within about15 hours such as, e.g., within about 12 hours, within about 10 hours, within 8 hours or within about 6 hours, when tested in an in vitro dissolution test and employing a dissolution medium comprising a buffer having pH 7.5
iv) at least about 55% w/w such as, e.g., about 60% w/w or more, about 65% w/w or more, about 70% w/w or more, about 75% w/w or more or about 80% w/w or more of the total amount of lercanidipine or an analogue thereof is released within about 5 hours such as, e.g., within about 4 hours, within about 3 hours, within about 2 hours, within about 1 hours or within about 30 minutes, when tested in an in vitro dissolution test and employing a dissolution medium comprising a buffer having pH 7.5, and/or
v) at least about 20% w/w such as, e.g., at least about 25% w/w, at least about 30% w/w, at least about 35% w/w or at least about 40% w/w of the total amount of lercanidipine or an analogue thereof is released within the first 3 hours such as, e.g., within the first 2 hours or within the first hour when tested in an in vitro dissolution test and employing a dissolution medium comprising a buffer having pH 7.5.

In an interesting embodiment, the composition is designed to have a delayed release of lercanidipine. Therefore, the invention also includes a pharmaceutical composition comprising lercanidipine together with one or more pharmaceutically acceptable excipient, wherein the composition upon oral administration to a mammal in need thereof has a prolonged/delayed release of lercanidipine so that at the most 10% w/w such as, e.g., at the most about 7.5% w/w or at the most about 5% w/w of the total amount of lercanidipine or an analogue thereof is released within the first two hours such as, e.g., within the first hour after administration.

In other embodiments, the following conditions are fulfilled with respect to *in vitro* dissolution test performed:
i) at the most about 30% w/w such as, e.g., at the most about 25% w/w, at the most about 20% w/w, at the most about 15% w/w or at the most about 10% w/w of lercanidipine or an analogue thereof is released within 2 hours in an in vitro dissolution test employing a dissolution medium having a pH of at the most about 5 such as, e.g. at the most about 4.5, at the most about 4, at the most about 3.5, at the most about 3, at the most about 2 or at the most about 1.5,
ii) at the most about 10% w/w such as, e.g., at the most about 7.5% w/w, at the most about 5% w/w or at the most about 2.5% w/w of lercanidipine or an analogue thereof is released within 2 hours in an in vitro dissolution test employing a dissolution medium having a pH of at the most about 5 such as, e.g. at the most about 4.5, at the most about 4, at the most about 3.5, at the most about 3, at the most about 2 or at the most about 1.5
iii) at the most about 60% w/w such as, e.g., at the most about 50% w/w, at the most about 40% w/w or at the most about 30% w/w of lercanidipine or an analogue thereof is released within 15 hours such as, e.g., within about 12 hours, when tested in an in vitro dissolution test employing a dissolution medium having a pH of at the most about 4.5 such as, e.g. at the most about 4.0, at the most about 3.5, at the most about 3, at the most about 2 or at the most about 1.5
iv) at the most about 40% w/w such as, e.g., at the most about 30% w/w, at the most about 25% w/w or at the most about 20% w/w of lercanidipine or an analogue thereof is released within 6 hours when tested in an in vitro dissolution test employing a dissolution medium having a pH of at the most about 4.5 such as, e.g. at the most about 4.0, at the most about 3.5, at the most about 3, at the most about 2 or at the most about 1.5, and/or
v) at the most about 30% w/w such as, e.g., at the most about 25% w/w, at the most about 20% w/w or at the most about 15% w/w of lercanidipine or an analogue thereof is released within 4 hours when tested in an in vitro dissolution test employing a dissolution medium having a pH of at the most about 4.5 such as, e.g. at the most about 4.0, at the most about 3.5, at the most about 3, at the most about 2 or at the most about 1.5.

The pharmaceutical compositions may be prepared by any convenient method such as, e.g. granulation, mixing, spray drying etc. A particularly useful method is the method described in WO 03/004001. Herein is described a process for the preparation of particulate material by a controlled agglomeration method, i. e. a method, which enables a controlled growth in particle size. The method involves spraying a first composition comprising e. g. lercanidipine and the vehicle, which has been melted, onto a solid carrier medium.

Preferably the melting point is at least 20°C and not higher than about 250°C.

In the present context, suitable carriers are e. g. those mentioned as an oily material (as discussed later herein) as well as those disclosed in WO 03/004001.

An advantage of using the controlled agglomeration method described in WO 03/004001 is that it is possible to apply a relatively large amount of a melt to a particulate material without having an undesirable growth in particle size. Accordingly, in one embodiment of the invention, the particulate material of a pharmaceutical composition has a geometric weight mean diameter d_{gw} of ≥ 10 µm such as, e.g. ≥ 20 pm, from about 20 to about 2000, from about 30 to about 2000, from about 50 to about 2000, from about 60 to about 2000, from about 75 to about 2000 such as, e. g. from about 100 to about 1500 µm, from about 100 to about 1000 µm or from about 100 to about 700 µm, or at the most about 400 µm or at the most 300 µm such as, e. g. , from about 50 to about 400 µm such as, e. g., from about 50 to about 350 µm, from about 50 to about 300 µm, from about 50 to about 250 µm or from about 100 to about 300 µm.

In another embodiment of this invention there is provided pharmaceutical compositions and solid dosage forms for improved treatment of conditions that respond to lercanidipine therapy and to combinations of lercanidipine and other anti hypertension, anti ischemic, anti diabetes, anti obesity, and cholesterol-or lipid-lowering agents. In one embodiment, the invention relates to pharmaceutical compositions comprising lercanidipine with one or more drugs of the above mentioned classes of therapeutic agents, for example lercanidipine in combination with carbonic anhydrase inhibitors like acetazolamide, diclorphenamide, methazolamide ; loop diuretics, like furosemide, bumetanide, ethacrynic acid, azosemide, muzolimine, piretanide, tripamide and torsemide etc.; inhibitors of Na and K symport, like hydrochlorthiazide, bendroflumethiazide, chlorothiazide, hydroflumethazide, methyclothiazide, polythiazide, triclormethazide, chlorthalidone, indapamide, metolazone, quinethazone etc.; inhibitors of renal ephitelial Na channels, like amiloride, triamterene; mineralocorticoids, like spironolactone, canrenone, potassium canrenoate etc.; angiotensin inhibitors like, losartan, candesartan, irbesartan, iosartan, eprosartan, telmisartan and valsartan etc.; ACE inhibitors like, trandolapril, ramipril, lisinopril, benazepril, captopril,enalapril, enalaprilat, fosinopril, moexipril, quinapril, perindopril, etc.; Ca²⁺ channel blockers like amlodipine, bepridil, diltiazem, felodipine, isradipine Nifedipine, nicardipine, nimodipine, nisoldipine, verapamil etc.; sympatolytic agents like Methyldopa, clonidine, guanabenz, guanfacine, guanadrel, reserpine, methyrosine; vasodilatators like hydrazine, minoxidil, nitroprusside, diazoxide; statins like, simvastatin, pravastatin, mevastatin, lovastatin, fluvastatin, atorvastatin, cerivastatin, etc.; fibrates like fenofibrate, clofibrate, gemfibrozil, bezafibrate and ciprofibrate; oral antidiabetics, like tolbutamide, chlorpropamide, tolazamide, acetohexamide, glyburide, glibenclamide, glipizide, gliclazide, glimiperide, replaglinide,nateglinide, metformin, rosiglitazone, pioglitazone; beta adrenergic receptor antagonists like, atenolol, nadolol, timolol, pindolol, labetalol, esmolol, acebutolol, bisoprolol, sotalol, propranolol, carvedilol, metoprolol etc.

### Pharmaceutically acceptable excipients

In the present context the terms "pharmaceutically acceptable excipient" are intended to denote any material, which is inert in the sense that it substantially does not have any therapeutic and/or prophylactic effect *per se.* Such an excipient may be added with the purpose of making it possible to obtain a pharmaceutical, cosmetic and/or foodstuff composition, which have acceptable technical properties.

Examples of suitable excipients for use in a composition or solid dosage form according to the invention include fillers, diluents, disintegrants, binders, lubricants etc. or mixture thereof. As the composition or solid dosage form according to the invention may be used for different purposes, the choice of excipients is normally made taken such different uses into considerations. Other pharmaceutically acceptable excipients for suitable use are e.g. acidifying agents, alkalizing agents, preservatives, antioxidants, buffering agents, chelating agents, coloring agents, complexing agents, emulsifying and/or solubilizing agents, flavors and perfumes, humectants, sweetening agents, wetting agents etc.

Examples of suitable fillers, diluents and/or binders include lactose (e.g. spray-dried lactose, α-lactose, β-lactose, Tabletose®, various grades of Pharmatose®, Microtose® or Fast-Floc®), microcrystalline cellulose (various grades of Avicel®, Elcema®, Vivacel®, Ming Tai® or Solka-Floc®), hydroxypropylcellulose, L-hydroxypropylcellulose (low substituted), hydroxypropyl methylcellulose (HPMC) (e.g. Methocel E, F and K, Metolose SH of Shin-Etsu, Ltd, such as, e.g. the 4,000 cps grades of Methocel E and Metolose 60 SH, the 4,000 cps grades of Methocel F and Metolose 65 SH, the 4,000, 15,000 and 100,000 cps grades of Methocel K; and the 4,000, 15,000, 39,000 and 100,000 grades of Metolose 90 SH), methylcellulose polymers (such as, e.g., Methocel A, Methocel A4C, Methocel A15C, Methocel A4M), hydroxyethylcellulose, sodium carboxymethylcellulose, carboxymethylene, carboxymethylhydroxyethylcellulose and other cellulose derivatives, sucrose, agarose, sorbitol, mannitol, dextrins, maltodextrins, starches or modified starches (including potato starch, maize starch and rice starch), calcium phosphate (e.g. basic calcium phosphate, calcium hydrogen phosphate, dicalcium phosphate hydrate), calcium sulfate, calcium carbonate, sodium alginate, collagen etc.

Specific examples of diluents are e.g. calcium carbonate, dibasic calcium phosphate, tribasic calcium phosphate, calcium sulfate, microcrystalline cellulose, powdered cellulose, dextrans, dextrin, dextrose, fructose, kaolin, lactose, mannitol, sorbitol, starch, pregelatinized starch, sucrose, sugar etc.

Specific examples of disintegrants are e.g. alginic acid or alginates, microcrystalline cellulose, hydroxypropyl cellulose and other cellulose derivatives, croscarmellose sodium, crospovidone, polacrillin potassium, sodium starch glycolate, starch, pregelatinized starch, carboxymethyl starch (e.g. Primogel® and Explotab®) etc.

Specific examples of binders are e.g. acacia, alginic acid, agar, calcium carrageenan, sodium carboxymethylcellulose, microcrystalline cellulose, dextrin, ethylcellulose, gelatin, liquid glucose, guar gum, hydroxypropyl methylcellulose, methylcellulose, pectin, PEG, povidone, pregelatinized starch etc.

Glidants and lubricants may also be included in the composition. Examples include stearic acid, magnesium stearate, calcium stearate or other metallic stearate, talc, waxes and glycerides, light mineral oil, PEG, glyceryl behenate, colloidal silica, hydrogenated vegetable oils, corn starch, sodium stearyl fumarate, polyethylene glycols, alkyl sulfates, sodium benzoate, sodium acetate etc.

Other excipients which may be included in a composition or solid dosage form of the invention are e.g. flavoring agents, coloring agents, taste-masking agents, pH-adjusting agents, buffering agents, preservatives, stabilizing agents, anti-oxidants, wetting agents, humidity-adjusting agents, surface-active agents, suspending agents, absorption enhancing agents, agents for modified release etc.

Other additives in a composition or a solid dosage form according to the invention may be antioxidants like e.g. ascorbic acid, ascorbyl palmitate, butylated hydroxyanisole, butylated hydroxytoluene, hypophosphorous acid, monothioglycerol, potassium metabisulfite, propyl gallate, sodium formaldehylde sulfoxylate, sodium metabisulfite, sodium thiosulfate, sulfur dioxide, tocopherol, tocopherol acetate, tocopherol hemisuccinate, TPGS or other tocopherol derivatives, etc. The carrier composition may also contain e.g. stabilising agents. The concentration of an antioxidant and/or a stabilizing agent in the carrier composition is normally from about 0.1 % w/w to about 5% w/w.

A composition or solid dosage form according to the invention may also include one or more surfactants or substances having surface-active properties. It is contemplated that such substances are involved in the wetting of the slightly soluble active substance and thus, contributes to improved solubility characteristics of the active substance.

Examples on surfactants are given in the following.

Suitable excipients for use in a composition or a solid dosage form according to the invention are surfactants such as, e.g., hydrophobic and/or hydrophilic surfactants as those disclosed in WO 00/50007 in the name of Lipocine, Inc. Examples on suitable surfactants are
i) polyethoxylated fatty acids such as, e.g. fatty acid mono- or diesters of polyethylene glycol or mixtures thereof such as, e.g. mono - or diesters of polyethylene glycol with lauric acid, oleic acid, stearic acid, myristic acid, ricinoleic acid, and the polyethylene glycol may be selected from PEG 4, PEG 5, PEG 6, PEG 7, PEG 8, PEG 9, PEG 10, PEG 12, PEG 15, PEG 20, PEG 25, PEG 30, PEG 32, PEG 40, PEG 45, PEG 50, PEG 55, PEG 100, PEG 200, PEG 400, PEG 600, PEG 800, PEG 1000, PEG 2000, PEG 3000, PEG 4000, PEG 5000, PEG 6000, PEG 7000, PEG 8000, PEG 9000, PEG 1000, PEG 10,000, PEG 15,000, PEG 20,000, PEG 35,000,
ii) polyethylene glycol glycerol fatty acid esters, i.e. esters like the above-mentioned but in the form of glyceryl esters of the individual fatty acids;
iii) glycerol, propylene glycol, ethylene glycol, PEG or sorbitol esters with e.g. vegetable oils like e.g. hydrogenated castor oil, almond oil, palm kernel oil, castor oil, apricot kernel oil, olive oil, peanut oil, hydrogenated palm kernel oil and the like,
iv) polyglycerized fatty acids like e.g. polyglycerol stearate, polyglycerol oleate, polyglycerol ricinoleate, polyglycerol linoleate,
v) propylene glycol fatty acid esters such as, e.g. propylene glycol monolaurate, propylene glycol ricinoleate and the like,
vi) mono- and diglycerides like e.g. glyceryl monooleate, glyceryl dioleae, glyceryl mono- and/or dioleate, glyceryl caprylate, glyceryl caprate etc.;
vii) sterol and sterol derivatives;
viii) polyethylene glycol sorbitan fatty acid esters (PEG-sorbitan fatty acid esters) such as esters of PEG with the various molecular weights indicated above, and the various Tween ® series (from ICI America, Inc.);
ix) polyethylene glycol alkyl ethers such as, e.g. PEG oleyl ether and PEG lauryl ether;
x) sugar esters like e.g. sucrose monopalmitate and sucrose monolaurate;
xi) polyethylene glycol alkyl phenols like e.g. the Triton® X or N series (from Union Carbide Chemicals & Plastics Technology Corporation);
xii) polyoxyethylene-polyoxypropylene block copolymers such as, e.g., the Pluronic® series from BASF Aktiengesellschaft, the Synperonic® series from ICI America, Inc., Emkalyx®, Lutrol® from BASF Aktiengesellschaft, Supronic® etc. The generic term for these polymers is "poloxamers" and relevant examples in the present context are Poloxamer 105, 108, 122, 123, 124, 181, 182, 183, 184, 185, 188, 212, 215, 217, 231, 234, 235, 237, 238, 282, 284, 288, 331, 333, 334, 335, 338, 401, 402, 403 and 407;
xiii) sorbitan fatty acid esters like the Span® series (from ICI) or Ariacel® series (from ICI) such as, e.g. sorbitan monolaurate, sorbitan monopalmitate, sorbitan monooleate, sorbitan monostearate etc.;
xiv) lower alcohol fatty acid esters like e.g. oleate, isopropyl myristate, isopropyl palmitate etc.;
xv) ionic surfactants including cationic, anionic and zwitterionic surfactants such as, e.g. fatty acid salts, bile salts, phospholipids, phosphoric acid esters, carboxylates, sulfates and sulfonates etc.

When a surfactant or a mixture of surfactants is present in a composition or a solid dosage form of the invention, the concentration of the surfactant(s) is normally in a range of from about 0,1 - 80% w/w such as, e.g., from about 0.1 to about 20% w/w, from about 0.1 to about 15% w/w, from about 0.5 to about 10% w/w, or alternatively, from about 0.10 to about 80% w/w such as, e.g. from about 10 to about 70% w/w, from about 20 to about 60% w/w or from about 30 to about 50% w/w.

In a specific aspect of the invention, at least one of the one or more pharmaceutically acceptable excipient is selected from the group consisting of silica acid or a derivative or salt thereof including silicates, silicon dioxide and polymers thereof; magnesium aluminosilicate and/or magnesium aluminometasilicate, bentonite, kaolin, magnesium trisilicate, montmorillonite and/or saponite.

Such materials are is especially useful as a sorption material for oils or oily-like materials in pharmaceuticals, cosmetics and/or foodstuff. In a specific embodiment, the material is used as a sorption material for oils or oily-like materials in pharmaceuticals. The material that has the ability to function as a sorption material for oils or oily-like materials is also denoted "oil sorption material". Furthermore, in the present context the term "sorption" is used to denote "absorption" as well as "adsorption". It should be understood that whenever one of the terms is used it is intended to cover the phenomenon absorption as well as adsorption.

Notably, the pharmaceutically acceptable excipient may comprise a silica acid or a derivative or salt thereof such as, e.g., silicon dioxide or a polymer thereof as a pharmaceutically acceptable excipient. Dependent on the quality employed a silicon dioxide may be a lubricant or it may be an oil sorption material. Qualities fulfilling the latter function seem to be most important.

In a specific embodiment, a composition or solid dosage form according to invention comprises a pharmaceutically acceptable excipient that is a silicon dioxide product that has properties corresponding to Zeofree® 5161A, Zeofree® 5162, Zeofree® 5175A, Zeopharm® 80 (available from J. M. Huber, Hamina, Finland), Aeroperl® 300, Sident® 22S, Sipernat®160, Sipernat® 160PQ, Sipernat® 22, Sipernat® 22 LS, Sipernat® 22, Sipernat® 22 LS, Sipernat® 22S, Sipernat® 2200, Sipernat® 310, Sipernat® 320, Sipernat® 320 DS, Sipernat® 325 C, Sipernat® 35, Sipernat® 350, Sipernat® 360, Sipernat® 383 D8, Sipernat® 44, Sipernat® 44MS, Sipernat® 50, Sipernat® 50S, Sipernat® 50 S, Sipernat® 500 LS, or Sipernat® 570 (available from Degussa, Frankfurt, Germany).

As it appears from the examples herein, a very suitable material is Aeroperl® 300 (including materials with properties like or corresponding to those of Aeroperl® 300).

Use of an oil sorption material in compositions or dosage forms according to the invention is very advantageous for the preparation of pharmaceutical, cosmetic, nutritional and/or food compositions, wherein the composition comprises oil or an oily-like material. One of the advantages is that is it possible to incorporate a relatively large amount of oil and oily-like material and still have a material that is solid. Thus, it is possible to prepare solid compositions with a relatively high load of oil or oily-like materials by use of an oil sorption material according to the invention. Within the pharmaceutical field it is an advantage to be able to incorporate a relatively large amount of an oil or an oily-like material in a solid composition especially in those situation where the active substance does not have suitable properties with respect to water solubility (e.g. poor water solubility), stability in aqueous medium (i.e. degradation occurs in aqueous medium), oral bioavailability (e.g. low bioavailability) etc., or in those situations where it is desired to modify the release of an active substance from a composition in order to obtain a controlled, delayed, sustained and/or pulsed delivery of the active substance. Thus, in a specific embodiment it is used in the preparation of pharmaceutical compositions.

The oil sorption material for use in the processing into solid compositions normally absorbs about 5% w/w or more, such as, e.g., about 10% w/w or more, about 15% w/w or more, about 20% w/w or more, about 25% w/w or more, about 30% w/w or more, about 35% w/w or more, about 40% w/w or more, about 45% w/w or more, about 50 w/w or more, about 55% w/w or more, about 60% w/w or more, about 65% w/w or more, about 70% w/w or more, about 75% w/w or more, about 80% w/w or more, about 85% w/w or more, about 90% w/w or more or about 95% w/w or more of an oil or an oily material and is still a solid material.

An important aspect of the invention is compositions or solid dosage forms comprising an oily material.

### Oily materials

In the present context the term "oily materials" is used in a very broad sense including oils, waxes, semi-solid materials and materials that normally are used as solvents (such as organic solvents) or co-solvents within the pharmaceutical industry, and the term also includes therapeutically and/or prophylactically active substances that are in liquid form at ambient temperature; furthermore the term includes emulsions like e.g. microemulsions and nanoemulsions and suspensions. The oily materials that can be absorbed are normally liquid at ambient or elevated temperature (for practical reasons the max. temperature is about 250 °C). They may be hydrophilic, lipophilic, hydrophobic and/or amphiphilic materials.

The oily material that are suitable for use in the present context are substances or materials, which have a melting point of at least about 0 °C and at the most about 250 °C.

In specific embodiments of the invention, the oil or oily-like material has a melting point of about 5°C or more such as, e.g., about 10°C or more, about 15°C or more, about 20°C or more or about 25°C or more.

In further embodiments of the invention, the oily material has a melting point of at least about 20°C such as, e.g., at least about 30°C at least about 35°C or at least about 40°C. For practical reasons, the melting point may normally not be too high, thus, the oil or oily-like material normally has a melting point of at the most about 300°C such as, e.g., at the most about 250°C, at the most about 200°C, at the most about 150°C or at the most about 100°C. If the melting point is higher a relatively high temperature may promote e.g. oxidation or other kind of degradation of an active substance in those cases where e.g. a therapeutically and/or prophylactically active substance is included.

In the present context, the melting point is determined by DSC (Differential Scanning Calorimetry). The melting point is determined as the temperature at which the linear increase of the DSC curve intersects the temperature axis (see Fig. 1 for further details).

Interesting oily materials are generally substances, which are used in the manufacture of pharmaceuticals as so-called melt binders or solid solvents (in the form of solid dosage form), or as co-solvents or ingredients in pharmaceuticals for topical use.

It may be hydrophilic, hydrophobic and/or have surface-active properties. In general hydrophilic and/or hydrophobic oily materials are suitable for use in the manufacture of a pharmaceutical composition comprising a therapeutically and/or prophylactically active substance that has a relatively low aqueous solubility and/or when the release of the active substance from the pharmaceutical composition is designed to be immediate or non-modified. Hydrophobic oily materials, on the other hand, are normally used in the manufacture of a modified release pharmaceutical composition. The above-given considerations are simplified to illustrate general principles, but there are many cases where other combinations of oily materials and other purposes are relevant and, therefore, the examples above should not in any way limit the invention.

Typically, a suitable hydrophilic oily material is selected from the group consisting of: polyether glycols such as, e.g., polyethylene glycols, polypropylene glycols; polyoxyethylenes; polyoxypropylenes; poloxamers and mixtures thereof, or it may be selected from the group consisting of: xylitol, sorbitol, potassium sodium tartrate, sucrose tribehenate, glucose, rhamnose, lactitol, behenic acid, hydroquinon monomethyl ether, sodium acetate, ethyl fumarate, myristic acid, citric acid, Gelucire 50/13, other Gelucire types such as, e.g., Gelucire 44/14 etc., Gelucire 50/10, Gelucire 62/05, Sucro-ester 7, Sucro-ester 11, Sucro-ester 15, maltose, mannitol and mixtures thereof.

A suitable hydrophobic oily material may be selected from the group consisting of: straight chain saturated hydrocarbons, sorbitan esters, paraffins; fats and oils such as e.g., cacao butter, beef tallow, lard, polyether glycol esters; higher fatty acid such as, e.g. stearic acid, myristic acid, palmitic acid, higher alcohols such as, e.g., cetanol, stearyl alcohol, low melting point waxes such as, e.g., glyceryl monostearate, glyceryl monooleate, hydrogenated tallow, myristyl alcohol, stearyl alcohol, substituted and/or unsubstituted monoglycerides, substituted and/or unsubstituted diglycerides, substituted and/or unsubstituted triglycerides, yellow beeswax, white beeswax, carnauba wax, castor wax, japan wax, acetylate monoglycerides; NVP polymers, PVP polymers, acrylic polymers, or a mixture thereof.

In an interesting embodiment, the oily material is a polyethylene glycol having an average molecular weight in a range of from about 400 to about 35,000 such as, e.g., from about 800 to about 35,000, from about 1,000 to about 35,000 such as, e.g., polyethylene glycol 1,000, polyethylene glycol 2,000, polyethylene glycol 3,000, polyethylene glycol 4,000, polyethylene glycol 5,000, polyethylene glycol 6000, polyethylene glycol 7,000, polyethylene glycol 8,000, polyethylene glycol 9,000 polyethylene glycol 10,000, polyethylene glycol 15,000, polyethylene glycol 20,000, or polyethylene glycol 35,000. In certain situations polyethylene glycol may be employed with a molecular weight from about 35,000 to about 100,000.

In another interesting embodiment, the oily material is polyethylene oxide having a molecular weight of from about 2,000 to about 7,000,000 such as, e.g. from about 2,000 to about 100,000, from about 5,000 to about 75,000, from about 10,000 to about 60,000, from about 15,000 to about 50,000, from about 20,000 to about 40,000, from about 100,000 to about 7,000,000 such as, e.g., from about 100,000 to about 1,000,000, from about 100,000 to about 600,000, from about 100,000 to about 400,000 or from about 100,000 to about 300,000.

In another embodiment, the oily material is a poloxamer such as, e.g. Poloxamer 188, Poloxamer 237, Poloxamer 338 or Poloxamer 407 or other block copolymers of ethylene oxide and propylene oxide such as the Pluronic® (from BASF) and/or Tetronic® (from BASF) series. Suitable block copolymers of the Pluronic® series include polymers having a molecular weight of about 3,000 or more such as, e.g. from about 4,000 to about 20,000 and/or a viscosity (Brookfield) from about 200 to about 4,000 cps such as, e.g., from about 250 to about 3,000 cps. Suitable examples include Pluronic® F38, P65, P68LF, P75, F77, P84, P85, F87, F88, F98, P103, P104, P105, F108, P123, F123, F127, 10R8, 17R8, 25R5, 25R8 etc. Suitable block copolymers of the Tetronic® series include polymers having a molecular weight of about 8,000 or more such as, e.g., from about 9,000 to about 35,000 and/or a viscosity (Brookfield) of from about 500 to about 45,000 cps such as, e.g., from about 600 to about 40,000. The viscosities given above are determined at 60 °C for substances that are pastes at room temperature and at 77 °C for substances that are solids at room temperature.

The oily material may also be a sorbitan ester such as, e.g., sorbitan di-isostearate, sorbitan dioleate, sorbitan monolaurate, sorbitan monoisostearate, sorbitan monooleate, sorbitan monopalmitate, sorbitan monostearate, sorbitan sesqui-isostearate, sorbitan sesquioleate, sorbitan sesquistearate, sorbitan tri-isostearate, sorbitan trioleate, sorbitan tristearate or mixtures thereof.

The oily material may of course comprise a mixture of different oils or oily-like materials such as, e.g., a mixture of hydrophilic and/or hydrophobic materials.

Other suitable oily materials may be solvents or semi-solid excipients like, e.g. propylene glycol, polyglycolised glycerides including Gelucire 44/14, complex fatty materials of plant origin including theobroma oil, carnauba wax, vegetable oils like e.g. almond oil, coconut oil, corn oil, cottonseed oil, sesame oil, soya oil, olive oil, castor oil, palm kernels oil, peanut oil, rape oil, grape seed oil etc., hydrogenated vegetable oils such as, e.g. hydrogenated peanut oil, hydrogenated palm kernels oil, hydrogenated cottonseed oil, hydrogenated soya oil, hydrogenated castor oil, hydrogenated coconut oil; natural fatty materials of animal origin including beeswax, lanolin, fatty alcohols including cetyl, stearyl, lauric, myristic, palmitic, stearic fatty alcohols; esters including glycerol stearate, glycol stearate, ethyl oleate, isopropyl myristate; liquid interesterified semi-synthetic glycerides including Miglycol 810/812; amide or fatty acid alcolamides including stearamide ethanol, diethanolamide of fatty coconut acids, acetic acid esters of mono and di-glycerides, citric acid esters of mono and di-glycerides, lactic acid esters of mono and diglycerides, mono and di-glycerides, poly-glycerol esters of fatty acids, poly-glycerol poly-ricinoleate, propylene glycol esters of fatty acids, sorbitan monostearates, sorbitan tristearates, sodium stearoyl
lactylates, calcium stearyl lactylates, diacetyl tartaric acid esters of mono and di-glycerides etc.

Normally, a pharmaceutical composition or a solid dosage form according to the invention has a concentration of the oily material in the composition of about 5% w/w or more such as, e. g. , about 10% w/w or more, about 15% w/w or more, about 20% w/w or more, about 25% w/w or more, about 30% w/w or more, about 35% w/w or more, about 40% w/w or more, about 45% w/w or more, about 50 w/w or more, about 55% w/w or more, about 60% w/w or more, about 65% w/w or more, about 70% w/w or more, about 75% w/w or more, about 80% w/w or more, about 85% w/w or more, about 90% w/w or more or about 95% w/w or more.

In specific embodiments the concentration of the oily material in a composition or solid dosage form of the invention is in a range from about 20% to about 80% w/w such as, e. g., from about 25% to about 75% w/w.

One of the advantages is that is it possible to incorporate a relatively large amount of oily material and still have a material that is solid. Thus, it is possible to prepare solid compositions with a relatively high load of oily materials by use of an oil sorption material according to the invention. Within the pharmaceutical field it is an advantage to be able to incorporate a relatively large amount of an oil or an oily-like material in a solid composition especially in those situation where the active substance does not have suitable properties with respect to water solubility (e. g. poor water solubility), stability in aqueous medium (i. e. degradation occurs in aqueous medium), oral bioavailability (e. g. low bioavailability) etc. , or in those situations where it is desired to modify the release of an active substance from a composition in order to obtain a controlled, delayed, sustained and/or pulsed delivery of the active substance.

A further advantage is that the particulate material obtained is a free-flowing powder and therefore readily processable into e. g. solid dosage forms such as tablets, capsules or sachets. Normally, the particulate material has properties that are suitable in order to manufacture tablets by direct compression without addition of large amounts of further additives. A suitable test for test the flowability of the particulate material is the method described in Ph. Eur. and measuring the flow rate of the material out of a funnel with a nozzle (orifice) diameter of 10.0 mm.

In embodiments outside of the scope of the invention, at least a part of lercanidipine and/or an analogue thereof is present in the composition in the form of a solid dispersion including a molecular dispersion and a solid solution. Normally, 10% or more such as, e. g., 20% or more, 30% or more, 40% or more, 50% or more, 60% or more, 70% or more, 80% or more, 90% or more such as, e. g. , 95% or more or about 100% w/w of lercanidipine and/or an analogue thereof is present in the composition in the form of a solid dispersion.

A solid dispersion may be obtained in different ways e. g. by employing organic solvents or by dispersing or dissolving the active substance in another suitable medium (e. g. an oily material that is in liquid form at room temperature or at elevated temperatures).

### Description of a solid dispersion based on organic solvents

Solid dispersions (solvent method) are prepared by dissolving a physical mixture of the active substance (e. g. a drug substance) and the carrier in a common organic solvent, followed by evaporation of the solvent. The carrier is often a hydrophilic polymer. Suitable organic solvents include pharmaceutical acceptable solvent in which the active substance is soluble such as methanol, ethanol, methylene chloride, chloroform, ethylacetate, acetone or mixtures thereof.

Suitable water soluble carriers include polymers such as polyethylene glycol, poloxamers, polyoxyethylene stearates, poly-E-caprolactone, polyvinylpyrrolidone (PVP), polyvinylpyrrolidone-polyvinylacetate copolymer PVP-PVA (Kollidon VA64), poly-methacrylic polymers (Eudragit RS, Eudragit RL, Eudragit NE, Eudragit E) and polyvinyl alcohol (PVA), hydroxypropyl cellulose (HPC), hydroxypropyl methyl cellulose (HPMC), methyl cellulose, and poly (ethylene oxide) (PEO).

Polymers containing acidic functional groups may be suitable for solid dispersions, which release the active substance in a preferred pH range providing acceptable absorption in the intestines. Such polymers may be one or more selected from the group comprising hydroxypropyl methylcellulose phtalate (HMPCP), polyvinyl acetate phtalate (PVAP), hydroxypropylmethylcellulose acetate succinate (HPMCAS), alginate, carbomer, carboxymethylcellulose, methacrylic acid copolymer (Eudragit L, Eudragit S), shellac, cellulose acetate phthalate (CAP), starch glycolat, polacrylin, methyl cellulose acetate phtalate, hydroxypropyulcellulose acetate phthalate, cellulose acetate terephtahalate, cellulose acetate isophthalate and cellulose acetate trimellitate.

In relations to amounts of the active substance and the polymer in the solid dispersion, the weight ratio of active substance to polymer may be in a range of from about 3: 1 to about 1: 20. However, narrower ranges of from about 3: 1 to about 1: 5, such as, e. g., from about 1: 1 to about 1: 3 or about may also be used.

The solid dispersion is preferably formed by spray drying techniques, controlled agglomeration, freeze-drying or coating on carrier particles or any other solvent removal process. The dried product contains the active substance present in the form of a solid dispersion including a molecular dispersion and a solid solution.

As an alternative to the use of organic solvents the drug and polymer may be co- grinded or extruded at elevated temperatures (melt extrusion).

The pharmaceutical compositions comprising lercanidipine at least partly in form of a solid dispersion or solution may in principle be prepared using any suitable procedure for preparing pharmaceutical compositions known within the art.

Apart from using the organic solvent based method, solid dispersion or solid solutions of lercanidipine and/or an analogue thereof may be obtained by dispersing and/or dissolving lercanidipine in the carrier composition used in the controlled agglomeration method. Stabilizing agents etc. may be added in order to ensure the stability of the solid dispersion/solution.

In another aspect, the invention relates to a method for the preparation of a pharmaceutical composition according to the invention. In general, any suitable method within the pharmaceutical field may be employed. However, in order to enable incorporation of a relatively high amount of an oil or an oily-like material especially the method described in WO 03/004001 (by the same inventors) has proved satisfactory. Details concerning the method are given in the above-identified publication, as well as in the Examples herein. In short, the invention provide a process for preparing a particulate pharmaceutical material comprising lercanidipine which method comprises spraying a first composition in liquid form, said composition comprising a carrier and having a melting point greater than 5°C onto a second composition comprising a support, said second composition being in the fluidised state and having a temperature less than the melting point of the carrier. In principle the active substance may be present in the carrier composition and/or in the second composition. However, in those cases where lercanidipine should be present in the composition at least partly as a solid dispersion, it is advantageous to incorporate or dissolve lercanidipine in the carrier composition.

### Solid dosage forms

A pharmaceutical composition according to the invention is in particulate form and may be employed as such. However, in many cases it is more convenient to present the composition in the form of granules, pellets, microspheres, nanoparticles and the like or in the form of solid dosage forms including tablets, capsules and sachets and the like.

A solid dosage form according to the invention may be a single unit dosage form or it may in the form of a polydepot dosage form contain a multiplicity of individual units such as, e.g., pellets, beads and/or granules.

Normally, a pharmaceutical composition or a solid dosage form of the invention is intended for administration via the oral, buccal or sublingual administration route.

The invention also relates to the above-mentioned presentation form. Within the scope of the invention are compositions/solid dosage forms that are intended to release lercanidipine in a fast release, a delayed release or modified release manner.

A solid dosage form according to the present invention comprises a pharmaceutical composition in particulate form as described above. The details and particulars disclosed under this main aspect of the invention apply *mutatis mutandis* to the other aspects of the invention. Accordingly, the properties with respect to increase in bioavailability, changes in bioavailability parameters, reduction in adverse food effect as well as release of lercanidipine etc. described and/or claimed herein for pharmaceutical compositions in particulate form are analogues for a solid dosage form according to the present invention.

Normally, the concentration of the pharmaceutical composition in particulate form is in a range of from about 5 to 100% w/w such as, e.g., from about 10% to about 90% w/w, from about 15% to about 85% w/w, from about 20% to about 80% w/w, from about 25% to about 80% w/w, from about 30% to about 80% w/w, from about 35% to about 80% w/w, from about 40% to about 75% w/w, from about 45% to about 75% w/w or from about 50% to about 70% w/w of the dosage form. In an embodiment of the invention, the concentration of the pharmaceutical composition in particulate form is 50% w/w or more of the dosage form.

A solid dosage form according to the invention is obtained by processing the particulate material according to the invention by means of techniques well-known to a person skilled in the art. Normally, it involves further addition of one or more of the pharmaceutically acceptable excipients mentioned herein.

The composition or solid dosage form according to the invention may be designed to release lercanidipine in any suitable manner provided that the increase in bioavailability is present. Thus, the active substance may be released relatively fast in order to obtain an enhanced on-set of action, it may be released so as to follow zero or first order kinetics or it may be released in a controlled or modified manner in order to obtain a predetermined pattern of release. Plain formulations are also within the scope of the present invention.

The composition or solid dosage form according to the invention may also be coated with a film coating, an enteric coating, a modified release coating, a protective coating, an anti-adhesive coating etc.

A solid dosage form according to the invention may also be coated in order to obtain suitable properties e.g. with respect to release of the active substance.

The coating may be applied on single unit dosage forms (e.g. tablets, capsules) or it may be applied on a polydepot dosage form or on its individual units.

Suitable coating materials are e.g. methylcellulose, hydroxypropylmethylcellulose, hydroxypropylcellulose, acrylic polymers, ethylcellulose, cellulose acetate phthalate, polyvinyl acetate phthalate, hydroxypropyl methylcellulose phthalate, polyvinylalcohol, sodium carboxymethylcellulose, cellulose acetate, cellulose acetate phthalate, gelatin, methacrylic acid copolymer, polyethylene glycol, shellac, sucrose, titanium dioxide, carnauba wax, microcrystalline wax, zein.

Plasticizers and other ingredients may be added in the coating material. The same or different active substance may also be added in the coating material.

In the following is given a more detailed description of interesting embodiments of the invention, i.e. embodiments wherein the solid dosage forms are designed to release the active substance and/or an analogue thereof in a controlled manner. In the present context, the term "controlled manner" is intended to include all types of release which differ from the release obtained from plain tablets. Thus, the term includes so-called "controlled release", "modified release", "sustained release", "pulsed release", "prolonged release", burst release", "slow release", "extended release", as well as the terms "delayed release" and pH dependant release. However, a specific aspect of the invention relates to a delayed release composition or dosage form, which in this context is intended to denote a composition or dosage form that at the most releases 10% w/w of the active substance within the first 2 hours after administration and/or after start of a dissolution test employing a dissolution medium having a pH of at the most about 3.

### Types of modified release systems

A first class includes matrix systems, in which lercanidipine is embedded or dispersed in a matrix of another material that serves to retard the release of lercanidipine into an aqueous environment (i.e., the luminal fluid of the GI tract). When lercanidipine is dispersed in a matrix of this sort, release of the drug takes place principally from the surface of the matrix. Thus the drug is released from the surface of a device, which incorporates the matrix after it diffuses through the matrix or when the surface of the device erodes, exposing the drug. In some embodiments, both mechanisms can operate simultaneously. The matrix systems may be large, i.e., tablet sized (about 1 cm), or small (< 0.3cm). The system may be unitary (e.g., a bolus), may be divided by virtue of being composed of several sub-units (for example, several capsules which constitute a single dose) which are administered substantially simultaneously, or may comprise a plurality of particles, also denoted a multiparticulate. A multiparticulate can have numerous formulation applications. For example, a multiparticulate may be used as a powder for filling a capsule shell, or used *per se* for mixing with food to increase palatability.

In a specific embodiment, a matrix multiparticulate, comprises a plurality of lercanidipine-containing particles, each particle comprising lercanidipine and/or an analogue thereof e.g. in the form of a solid dispersion with one or more excipients selected to form a matrix capable of controlling the dissolution rate of the lercanidipine into an aqueous medium. The matrix materials useful for this embodiment are generally water-insoluble materials such as waxes, cellulose, or other water-insoluble polymers. If needed, the matrix materials may optionally be formulated with water-soluble materials, which can be used as binders or as enhancers. Matrix materials useful for the manufacture of these dosage forms such as: Hydroxypropyl methyl cellulose, waxes such as paraffin, modified vegetable oils, camauba wax, hydrogenated castor oil, beeswax, and the like, as well as synthetic polymers such as poly(vinyl chloride), poly(vinyl acetate), copolymers of vinyl acetate and ethylene, polystyrene, and the like. Water soluble binders or release modifying agents which can optionally be formulated into the matrix include water-soluble polymers such as hydroxypropyl cellulose (HPC), hydroxypropyl methyl cellulose (HPMC), methyl cellulose, poly (N-vinyl-2-pyrrolidinone) (PVP), poly(ethylene oxide) (PEO), poly(vinyl alcohol) (PVA), xanthan gum, carrageenan, and other such natural and synthetic materials. In addition, materials, which function as release-modifying agents include water-soluble materials such as sugars or salts. Preferred water-soluble materials include lactose, sucrose, glucose, and mannitol, as well as HPC, HPMC, and PVP.

In a specific embodiment, a multiparticulate product is defined as being processed by controlled agglomeration. In this case lercanidipine is dispersed in a suitable meltable carrier and sprayed on carrier particles comprising the matrix substance. Alternatively, lercanidipine is dispersed in an organic solvent together with the matrix substance and spray dried or applied to carrier particles.

Solvents typically employed for the process include acetone, ethanol, isopropanol, ethyl acetate, and mixtures of two or more (for further details reference is given to the paragraphs under the heading Description of a solid dispersion based on organic solvents).

Once formed, lercanidipine matrix multiparticulates may be blended with compressible excipients such as lactose, microcrystalline cellulose, dicalcium phosphate, and the like and the blend compressed to form a tablet. Disintegrants such as sodium starch glycolate or crosslinked poly(vinyl pyrrolidone) are also usefully employed. Tablets prepared by this method disintegrate when placed in an aqueous medium (such as the GI tract), thereby exposing the multiparticulate matrix, which releases lercanidipine therefrom.

A further embodiment of a matrix system has the form of a hydrophilic matrix tablet containing lercanidipine and/or an analogue thereof (e.g. in the form of a solid dispersion) as a multiparticulate product and an amount of hydrophilic polymer sufficient to provide a useful degree of control over the lercanidipine dissolution. Hydrophilic polymers useful for forming the matrix include hydroxypropylmethyl cellulose (HPMC), hydroxypropyl cellulose (HPC), poly (ethylene oxide), poly(vinyl alcohol), xanthan gum, carbomer, carrageenan, and zooglan. A preferred material is HPMC. Other similar hydrophilic polymers may also be employed. In use, the hydrophilic material is swollen by, and eventually dissolves in, water. The lercanidipine is released both by diffusion from the matrix and by erosion of the matrix. The lercanidipine dissolution rate of these hydrophilic matrix tablets may be controlled by the amount and molecular weight of hydrophilic polymer employed. In general, using a greater amount of the hydrophilic polymer decreases the dissolution rate, as does using a higher molecular weight polymer. Using a lower molecular weight polymer increases the dissolution rate. The dissolution rate may also be controlled by the use of water-soluble additives such as sugars, salts, or soluble polymers. Examples of these additives are sugars such as lactose, sucrose, or mannitol, salts such as NaCl, KCl, NaHCO₃, and water soluble polymers such as PNVP or PVP, low molecular weight HPC or HMPC or methyl cellulose. In general, increasing the fraction of soluble material in the formulation increases the release rate. A matrix tablet typically comprises about 20 to 90% by weight of lercanidipine and about 80 to 10% by weight of polymer.

A preferred matrix tablet comprises, by weight, about 30% to about 80% solid dispersion containing lercanidipine and/or an analogue thereof about 15% to about 35% matrix former (such as, e.g., HPMC), 0% to about 35% lactose, 0% to about 20% microcrystalline cellulose, and about 0.25% to about 2% lubricant (such as, e.g., magnesium stearate).

The matrix systems as a class often exhibit non-constant release of the drug from the matrix. This result may be a consequence of the diffusive mechanism of drug release, and modifications to the geometry of the dosage form can be used to advantage to make the release rate of the drug more constant.

A second class of lercanidipine sustained-release dosage forms of this invention includes membrane-moderated or reservoir systems. In this class, a reservoir of lercanidipine e.g. in a solid dispersion as a multiparticulate product is surrounded by a rate-limiting membrane. The lercanidipine traverses the membrane by mass transport mechanisms well known in the art, including but not limited to dissolution in the membrane followed by diffusion across the membrane or diffusion through liquid-filled pores within the membrane. These individual reservoir system dosage forms may be large, as in the case of a tablet containing a single large reservoir, or multiparticulate, as in the case of a capsule or polydepot tablets containing a plurality of reservoir particles, each individually coated with a membrane. The coating can be non-porous, yet permeable to lercanidipine (for example lercanidipine may diffuse directly through the membrane), or it may be porous. As with other embodiments of this invention, the particular mechanism of transport is not believed to be critical.

Sustained release coatings as known in the art may be employed to fabricate the membrane, especially polymer coatings, such as a cellulose ester or ether, an acrylic polymer, or a mixture of polymers. Preferred materials include ethyl cellulose, cellulose acetate and cellulose acetate butyrate. The polymer may be applied as a solution in an organic solvent or as an aqueous dispersion or latex. The coating operation may be conducted in standard equipment such as a fluid bed coater, a Wurster coater, or a rotary fluid bed coater.

If desired, the permeability of the coating may be adjusted by blending of two or more materials. A particularly useful process for tailoring the porosity of the coating comprises adding a pre-determined amount of a finely-divided water-soluble material, such as sugars or salts or water-soluble polymers to a solution or dispersion (e.g., an aqueous latex) of the membrane-forming polymer to be used. When the dosage form is ingested into the aqueous medium of the GI tract, these water soluble membrane additives are leached out of the membrane, leaving pores which facilitate release of the drug. The membrane coating can also be modified by the addition of plasticizers, as known in the art.

A particularly useful variation of the process for applying a membrane coating comprises dissolving the coating polymer in a mixture of solvents chosen such that as the coating dries, a phase inversion takes place in the applied coating solution, resulting in a membrane with a porous structure.

In general, a support for mechanically strengthening the membrane is not required.

The morphology of the membrane is not of critical importance so long as the permeability characteristics enumerated herein are met. The membrane can be amorphous or crystalline. It can have any category of morphology produced by any particular process and can be, for example, an interfacially-polymerized membrane (which comprises a thin rate-limiting skin on a porous support), a porous hydrophilic membrane, a porous hydrophobic membrane, a hydrogel membrane, an ionic membrane, and other such materials which are characterized by controlled permeability to lercanidipine.

A sustained release coating as known in the art, especially polymer coatings, may be employed to fabricate the membrane. Suitable and preferred polymer coating materials, equipment, and coating methods also include those previously discussed.

The rate of lercanidipine release from the coated multiparticulates can also be controlled by factors such as the composition and binder content of the drug-containing core, the thickness and permeability of the coating, and the surface-to-volume ratio of the multiparticulates. It will be appreciated by those skilled in the art that increasing the thickness of the coating will decrease the release rate, whereas increasing the permeability of the coating or the surface-to-volume ratio of the multiparticulates will increase the release rate. If desired, the permeability of the coating may be adjusted by blending of two or more materials. A useful series of coatings comprises mixtures of water-insoluble and water-soluble polymers, for example, ethylcellulose and hydroxypropyl methylcellulose, respectively. A particularly useful modification to the coating is the addition of finely divided water-soluble material, such as sugars or salts. When placed in an aqueous medium, these water soluble membrane additives are leached out of the membrane, leaving pores which facilitate delivery of the drug. The membrane coating may also be modified by the addition of plasticizers, as is known to those skilled in the art.

In one embodiment of the invention it is an aim to reduce the exposure of the upper GI tract to high concentrations of lercanidipine. Accordingly, suitable dosage forms includes those forms, which incorporate a delay before the onset of sustained release of lercanidipine. An exemplary embodiment can be illustrated by a tablet (or a particulate material) comprising a core containing lercanidipine coated with a first coating of a polymeric material of the type useful for sustained release of lercanidipine and a second coating of the type useful for delaying release of drugs when the dosage form is ingested. The first coating is applied over and surrounds the tablet or individual particles. The second coating is applied over and surrounds the first coating.

A tablet can be prepared by techniques well known in the art and contains a therapeutically useful amount of lercanidipine plus such excipients as are necessary to form the tablet by such techniques.

The first coating may be a sustained release coating as known in the art, especially polymer coatings, to fabricate the membrane, as previously discussed for reservoir systems. Suitable and preferred polymer coating materials, equipment, and coating methods also include those previously discussed.

Materials useful for preparing the second coating on the tablet include polymers known in the art as enteric coatings for delayed-release of pharmaceuticals. These most commonly are pH-sensitive materials such as cellulose acetate phthalate, cellulose acetate trimellitate, hydroxypropyl methyl cellulose phthalate, poly (vinyl acetate phthalate), and acrylic copolymers such as Eudragit L-100 (Röhm Pharma) and related materials, as more fully detailed below under "Delayed Release". The thickness of the delayed-release coating is adjusted to give the desired delay property. In general, thicker coatings are more resistant to erosion and, consequently, yield a longer delay. Preferred coatings range from about 300 µm in thickness to about 3 mm in thickness.

When ingested, the twice-coated tablet passes through the stomach, where the second coating prevents release of the lercanidipine under the acidic conditions prevalent there. When the tablet passes out of the stomach and into the small intestine, where the pH is higher, the second coating erodes or dissolves according to the physicochemical properties of the chosen material. Upon erosion or dissolution of the second coating, the first coating prevents immediate or rapid release of the lercanidipine and modulates the release so as to prevent the production of high concentrations, thereby minimizing side-effects.

A further preferred embodiment comprises a multiparticulate wherein each particle is dual coated as described above for tablets, first with a polymer designed to yield sustained release of the lercanidipine and then coated with a polymer designed to delay onset of release in the environment of the GI tract when the dosage form is ingested.

The sustained release coating may be as known in the art, especially polymer coatings, to fabricate the membrane, as previously discussed for reservoir systems. Suitable and preferred polymer coating materials, equipment, and coating methods also include those previously discussed.

The rate of lercanidipine release from the sustained-release-coated multiparticulates (i.e., the multiparticulates before they receive the delayed-release coating) and methods of modifying the coating are also controlled by the factors previously discussed for reservoir system lercanidipine multiparticulates.

The second membrane or coating for dual coated multiparticulates is a delayed-release coating which is applied over the first sustained-release coating, as disclosed above for tablets, and may be formed from the same materials. It should be noted that the use of the so-called "enteric" materials to practice this embodiment differs significantly from their use to produce conventional enteric dosage forms. With conventional enteric forms, the object is to delay release of the drug until the dosage form has passed the stomach and then to deliver the dose in the duodenum. Dosing of lercanidipine directly and completely to the duodenum may be undesirable, however, due to the side effects sought to be minimized or avoided by this invention. Therefore, if conventional enteric polymers are to be used to practice this embodiment, it may be necessary to apply them significantly more thickly than in conventional practice, in order to delay drug release until the dosage form reaches the lower GI tract. However, it is also possible to effect a sustained or controlled delivery of lercanidipine after the delayed-release coating has dissolved or eroded, therefore the benefits of this embodiment may be realized with a proper combination of delayed-release character with sustained-release character, and the delayed-release part alone may or may not necessarily conform to USP enteric criteria. The thickness of the delayed-release coating is adjusted to give the desired delay property. In general, thicker coatings are more resistant to erosion and, consequently, yield a longer delay.

A first delayed release embodiment according to the invention is a "pH-dependent coated tablet", which comprises a tablet core comprising lercanidipine e.g. in a solid dispersion as a multiparticulate product, a disintegrant, a lubricant, and one or more pharmaceutical carriers, such core being coated with a material, preferably a polymer, which is substantially insoluble and impermeable at the pH of the stomach, and which is more soluble and permeable at the pH of the small intestine. Preferably, the coating polymer is substantially insoluble and impermeable at pH <5.0, and water-soluble at pH>5.0. The tablet core may be coated with an amount of polymer sufficient to assure that substantially no release of lercanidipine from the dosage form occurs until the dosage form has exited the stomach and has resided in the small intestine for about 15 minutes or greater, preferably about 30 minutes or greater, thus assuring that minimal lercanidipine is released in the duodenum. Mixtures of a pH-sensitive polymer with a water-insoluble polymer may also be employed. Tablets are coated with an amount of polymer comprising from about 10% to about 80% of the weight of the lercanidipine-containing tablet core. Preferred tablets are coated with an amount of polymer comprising about 15% to about 50% of the weight of the lercanidipine tablet core.

pH-sensitive polymers which are relatively insoluble and impermeable at the pH of the stomach, but which are more soluble and permeable at the pH of the small intestine and colon include polyacrylamides, phthalate derivatives such as acid phthalates of carbohydrates, amylose acetate phthalate, cellulose acetate phthalate, other cellulose ester phthalates, cellulose ether phthalates, hydroxypropylcellulose phthalate, hydroxypropylethylcellulose phthalate, hydroxypropylmethylcellulose phthalate, methylcellulose phthalate, polyvinyl acetate phthalate, polyvinyl acetate hydrogen phthalate, sodium cellulose acetate phthalate, starch acid phthalate, styrene-maleic acid dibutyl phthalate copolymer, styrene-maleic acid polyvinylacetate phthalate copolymer, styrene and maleic acid copolymers, polyacrylic acid derivatives such as acrylic acid and acrylic ester copolymers, polymethacrylic acid and esters thereof, poly acrylic methacrylic acid copolymers, shellac, and vinyl acetate and crotonic acid copolymers.

Preferred pH-sensitive polymers include shellac; phthalate derivatives, particularly cellulose acetate phthalate, polyvinylacetate phthalate, and hydroxypropylmethylcellulose phthalate; polyacrylic acid derivatives, particularly polymethyl methacrylate blended with acrylic acid and acrylic ester copolymers; and vinyl acetate and crotonic acid copolymers.

Cellulose acetate phthalate (CAP) may be applied to lercanidipine tablets to provide delayed release of lercanidipine until the lercanidipine-containing tablet has passed the sensitive duodenal region, that is to delay the release of lercanidipine in the gastrointestinal tract until about 15 minutes, and preferably about 30 minutes, after the lercanidipine-containing tablet has passed from the stomach to the duodenum. The CAP coating solution may also contain one or more plasticizers, such as diethyl phthalate, polyethyleneglycol-400, triacetin, triacetin citrate, propylene glycol, and others as known in the art. Preferred plasticizers are diethyl phthalate and triacetin. The CAP coating formulation may also contain one or more emulsifiers, such as polysorbate-80.

Anionic acrylic copolymers of methacrylic acid and methylmethacrylate are also particularly useful coating materials for delaying the release of lercanidipine from lercanidipine-containing tablets until the tablets have moved to a position in the small intestine, which is distal to the duodenum. Copolymers of this type are available from RöhmPharma Corp, under the tradenames Eudragit-L® and Eudragit-S®. Eudragit-L® and Eudragit-S® are anionic copolymers of methacrylic acid and methylmethacrylate. The ratio of free carboxyl groups to the esters is approximately 1:1 in Eudragit-L® and approximately 1:2 in Eudragit-S®. Mixtures of Eudragit-L® and Eudragit-S® may also be used. For coating of lercanidipine-containing tablets, these acrylic coating polymers must be dissolved in an organic solvent or mixture of organic solvents. Useful solvents for this purpose are acetone, isopropyl alcohol, and methylene chloride. It is generally advisable to include 5-20% plasticizers in coating formulations of acrylic copolymers. Useful plasticizers are polyethylene glycols, propylene glycols, diethyl phthalate, dibutyl phthalate, castor oil, and triacetin.

The delay time before release of lercanidipine, after the "pH-dependent coated tablet" dosage form has exited the stomach, may be controlled by choice of the relative amounts of Eudragit-L® and Eudragit-S® in the coating, and by choice of the coating thickness. Eudragit-L® films dissolve above pH 6.0, and Eudragit-S® films dissolve above 7.0, and mixtures dissolve at intermediate pH's. Since the pH of the duodenum is approximately 6.0 and the pH of the colon is approximately 7.0, coatings composed of mixtures of Eudragit-L® and Eudragit-S® provide protection of the duodenum from lercanidipine. If it is desired to delay release of lercanidipine until the lercanidipine-containing "pH-dependent coated tablet" has reached the colon, Eudragit-S® may be used as the coating material, as described by Dew et al (Br. J. Clin. Pharmac. 14 (1982) 405-408). In order to delay the release of lercanidipine for about 15 minutes or more, preferably 30 minutes or more, after the dosage form has exited the stomach, preferred coatings comprise from about 9:1 to about 1:9 Eudragit-L® /Eudragit-S®, more preferably from about 9:1 to about 1:4 Eudragit-L® /Eudragit-S®. The coating may comprise from about 3% to about 70% of the weight of the uncoated tablet core. Preferably, the coating comprises from about 5% to about 50% of the weight of the tablet core.

The invention is further illustrated in the following examples without limiting it thereto.

### Materials and methods

### Materials

Glyceryl monocaprylate, Imwitor 308 from Sasol Germany GmbH, D-58453 Witten, Germany.
Glyceryl monolaurate, Dimodan ML 90/B or Rylo MG 12 (Ph.Eur.) from Danisco A/S, DK-1001 Copenhagen K, Denmark.
Polyglycolized glycerides, Gelucire® 44/14 from Gattefosse, F-69804 Saint-Priest, France.
Hypromellose (HPMC) & methylcellulose, Metolose™ 90SH 100cP or 15000cP, Shin-Etsu Chemical Co., Tokyo, Japan
Magnesium alumino(meta)silicate, Neusilin® US2 from Fuji Chemical Industry Co., Ltd., Toyama, Japan
Polyvinylpyrrolidone, Povidone K30 from Friends Union Enterprises Ltd., Tianjin, China Cellulose microcrystalline, Avicel PH102 from FMC BioPolymer, Cork, Ireland Magnesium stearate MF 2V from Unikem, Copenhagen, Denmark
Poloxamer 188, Lutrol® F68 from BASF, U.S.A.
NVP homopolymer, Kollidon® from BASF, D-67056 Ludwigshafen, Germany.
Lercanidipine, HCI from Recordati, Milan, Italy.

Tablets, capsules or granules might be enteric coated with different types of polymers such as hydroxypropylmethylcellulose acetate succinate (Aqoat), cellulose acetate phthalate CAP, hydroxypropylmethylcellulose phtalate HPMCP or methacrylic acid copolymers such as Eudragit L30D, Eudragit 100/S, Eudragit 100/L.

Zanidip® tablet formulation, see example 13.

### Equipment

Laboratory scale fluid bed equipment: Strea-1.

The melt feed unit is a prototype composed of separate units for heating of air supplies for the atomizer, pressure tank and feeding tube. Granulate was sieved manually and mixed with extragranular excipients in a Turbula mixer.

Tablet compression was performed on a single punch press, Diaf TM20.

### Methods

According to one method of the invention, the active substance was dissolved into the melted vehicle and applied on the particulate carrier(s) as follows:
The vehicle was melted in a beaker placed in a microwave oven. The beaker was transferred to a temperature controlled heating plate supplied with magnetic stirring. Active substance was dissolved slowly in the melt at a temperature of 60-105 °C under magnetic stirring. The hot solution was transferred to the pressure tank for melt spray application onto the carrier in the fluid bed. The granulate product was discharged from the fluid bed and sieved through sieve 0.7 mm or 1.0 mm manually. The sieved product was blended with magnesium stearate for 0.5 min in a Turbula mixer. If an extragranular phase has to be incorporated, the extragranular phase was premixed with the granulate in 3 minutes in a Turbula mixer.

The tablet compression was performed on a single punch machine Diaf TM20.

In another method of the invention, the active substance was dispersed in the vehicle following by homogenization in an Ultra-Turrax apparatus for 3 minutes. All other process steps are identical to the steps of the method for preparing granulate comprising active substance in a dissolved state.

For the preparation of a pharmaceutical composition in particulate form according to the invention, the method described in WO 03/004001 may be used. The method ensures a controlled agglomeration process, i.e. a strict control of the growth in particle size while at the same time it is possible to use a relatively large amount of an oily material.

### Determination of weight variation

The tablets prepared in the Examples herein were subject to a test for weight variation performed in accordance with Ph. Eur.

### Determination of average tablet hardness

The tablets prepared in the Examples herein were subject to at test for tablet hardness employing Schleuniger Model 6D apparatus and performed in accordance with the general instructions for the apparatus.

### Determination of disintegration time

The time for a tablet to disintegrate, i.e. to decompose into particles or agglomerates, was determined in accordance with Ph. Eur.

### Determination of geometric weight mean diameter d_{gw}

The geometric weight mean diameter was determined by employment of a method of laser diffraction dispersing the particulate material obtained (or the starting material) in air. The measurements were performed at 1 bar dispersive pressure in Sympatec Helos equipment, which records the distribution of the equivalent spherical diameter. This distribution is fitted to a log normal volume-size distribution.

When used herein, "geometric weight mean diameter" means the mean diameter of the log normal volume-size distribution.

### Determination of dissolution rate

The dissolution rate was determined by employment of Ph. Eur. 2.9.3 paddle dissolution test using 100 rpm and 900 mL of dissolution medium 0.3% polysorbate 80 in 0.1N HCl, 37 °C.

### Determination of solid solution

According to one embodiment of the present invention, lercanidipine is dissolved in a vehicle. In order to substantiate this, a test involving differential scanning calometry is performed. The test is performed on the particulate composition, solid dosage form or mixture of vehicle and lercanidipine (after the solid solution is supposed to form).

Standard DSC equipment connected to a PC is used.

| | |
|---|---|
| Sample size : | 10 mg in alu pans |
| Heating rate: | 5°C/min from 27°C to 110°C |
| Evaluation: | Lercanidipine is considered to be in dissolved state or non-crystalline if no lercanidipine endoterm peak is observed and if the melting interval does not significantly shift compared with the vehicle alone. |

### In vivo studies in Beagle dogs

In vivo studies with the purpose of determining the bioavailability of the compositions relative to the bioavailability of the commercially available fenofibrate tablet formulation, i.e. Zanidip®, was performed using Beagle dogs.

The experimental work was performed using four male Beagle dogs each having a body weight of 12-18 kg (starting weight). The studies were conducted as open, nonrandomised, cross-over studies. Each animal was its own control. Oral doses of lercanidipine were administered according to the data below. Each dog was dosed with the specified dose of lercanidipine without taking the weight of the dog into consideration.

Blood samples were collected at vena jugularis externa at the following points of time: Pre-dose, 1,1. 5,2, 3,4, 6,8, 12 and 24 hours after dosing. 4 ml of blood were collected, mixed with EDTA, and the samples were frozen (-80°C). The blood samples were analyzed using on-line extraction LC/MS and results were given in mg/mL.

The determined full blood concentration profiles of fenofibrate were treated using the Pharmacokinetic softwear WinNonlin®, (Pharsight, California; USA) to calculate the pharmacokinetic parameters. All data are dose adjusted, when necessary.

The following examples serve the purpose of illustration of the invention and are not intended to limiting the scope of the present invention. Pharmaceutical compositions and dosage forms of the invention are exemplified in examples 1-10. Examples 1, 2, 8, and 9 fall outside of the scope of the present invention. Results of in vitro dissolution tests of dosage forms of the invention are found in example 11. Results of stability tests of dosage forms of the invention are found in example 12. Results of in vivo comparison studies in Beagle dogs (blood plasma concentration) are found in example 13- 14.

### Example 1 (not according to the invention)

### Matrix capsules with intragranular hydrocolloid

**Capsule composition**

| Substance | % | mg |
|---|---|---|
| Lercanidipine HCl | 3.811 | 20.00 |
| Metolose HS 90 100 cP | 20.86 | 109.53 |
| Lactose 200 mesh | 29.39 | 154.30 |
| PEG 6000 | 32.15 | 168.78 |
| Poloxamer 188 | 13.78 | 72.33 |
| Total | 100.00 | 525.00 |

20 g lercanidipine was dissolved in a melted mixture of polyethylene glycol 6000 and Poloxamer 188 (70:30) at 90 °C. 318 g of the solid dispersion was sprayed on a mixture of 150 g of lactose and 100 g Metolose 90SH 100 cP in a fluid bed Strea-1. The granular product was sieved through sieve 0.7 mm. The granular product was sieved through sieve 0.7 mm and filled into hard gelatin capsules.

### Example 2 (not according to the invention)

### Matrix tablets with extragranular hydrocolloid

**Tablet composition**

| Substance | % | Mg |
|---|---|---|
| Lercanidipine HCl | 1.61 | 10.00 |
| Lactose 200 mesh | 38.14 | 237.5 |
| PEG 6000 | 27.83 | 173.3 |
| Poloxamer 188 | 11.93 | 74.3 |
| Metolose HS 90 15000 cP | 20.00 | 124.5 |
| Magnesium stearate | 0.5 | 3.1 |
| Total | 100.00 | 525.00 |

The granular product from Example 1 is mixed with 20% Metolose HS 90 15000 cP in a turbula mixer for 3 minutes and subsequently mixed with 0.5% magnesium stearate for 0.5 min. The granulate was directly compressed into 12 mm tablets (compound cup) on a Diaf TM20. The tablets had a mean weight of 623 mg and a strength of 10 mg. Mean tablet hardness: 51 N.

### Example 3

### Lercanidipine capsules

**Composition (Composition B):**

| Substance | Mg |
|---|---|
| Lercanidipine HCl | 20.00 |
| Glyceryl monocaprylate (Imwitor 308) | 180.0 |
| Total | 200.0 |

2.5 g lercanidipine was dissolved in 22.5 g glyceryl monocaprylate at about 100°C. The clear solution was filled in 200 mg capsules size 1 CS.

### Examples 4-5

### Tablets comprising a solid solution of lercanidipine

The following compositions were prepared:

| **Substance** | **Ingredient** | **Comp. C mg** | **Comp. D mg** |
|---|---|---|---|
| **Drug** | Lercanidipine HCl | 10.0 | 20.0 |
| **Vehicle** | Glyceryl monolaurate (Dimodan ML90/B) | 190.0 | 230.0 |
| **Carrier** | Magnesium aluminosilicate (Neusilin US2) | 57.7 | 138.8 |
| **Excipients** | Mg stearate | 4.4 | 8.8 |
| | Cellulose microcryst. (Avicel PH102) | 144.2 | - |
| | Hypromellose (Metolose 90SH100) | - | 44.2 |
| | Croscarmellose sodium (Ac-di-sol) | 30.6 | |
| **Total** | | 436.9 | 441.8 |

### Formulation C:

17.5 g of lercanidipine was dissolved in 332.5 g of glyceryl monolaurate at 105 °C. 350 g of the solid solution was sprayed on 110.0 g of magnesium aluminosilicate in a fluid bed Strea-1. The granular product was sieved through sieve 0.7 mm. The granular product was mixed with 176.8 g microcrystalline cellulose and 41.5 g of croscarmellose sodium in a turbula mixer for 3 minutes and subsequently mixed with 5.9 g magnesium stearate for 0.5 min. The granulate was directly compressed into 12 mm tablets (compound cup) on a Diaf TM20. The tablets had a mean weight of 434 mg and a strength of 10 mg.
Mean tablet hardness: 82N. Disintegration time: 20 minutes.

### Formulation D (not according to the invention):

The tablets were prepared using the same method as for formulation C using hypromellose as extragranular excipient. The granulate was directly compressed into 8 x 18 mm oblong tablets (compound cup) on a Diaf TM20. The tablets had a mean weight of 442 mg and strength 20 mg.
Mean tablet hardness: 59 N.

### Examples 6-7

### Tablets comprising a solid solution of lercanidipine

The following compositions were prepared:

| **Substance** | **Ingredient** | **Form. E Mg** | **Form. F mg** |
|---|---|---|---|
| **Drug** | Lercanidipine HCl | 10.0 | 10.0 |
| **Vehicle** | Glyceryl monocaprylate (Imwitor 308) | 90.0 | - |
| | Glyceryl monocaprate | - | 190.0 |
| **Carrier** | Magnesium aluminosilicate | 68.4 | 68.5 |
| **Excipients** | Mg stearate | 2.9 | 4.6 |
| | Cellulose microcryst. (Avicel PH102) | 135.2 | 182.0 |
| **Total** | | 285.4 | 455.1 |
| **Hardness** | N | 41 | 62 |
| **Disintegration time** | Minutes | - | - |
| **Diameter** | Mm | 9 | 11 |

### Formulation E:

21.5 g of lercanidipine was dissolved in 193.5 g of glyceryl monocaprylate at 95 °C. The solid solution was sprayed on 130.0 g of magnesium aluminosilicate in a fluid bed Strea-1. The granular product was sieved through sieve 0.7 mm. The granular product was mixed with 135.6 g microcrystalline cellulose and in a turbula mixer for 3 minutes and subsequently mixed with 3.4 g of magnesium stearate for 0.5 min. The granulate was directly compressed into 9 mm tablets on a Diaf TM20. The tablets had a mean weight of 285 mg and strength 10 mg.
Mean tablet hardness: 41N.

### Formulation F:

20.5 g of lercanidipine was dissolved in 389.5 g of glyceryl monocaprate at 95 °C. The solid solution was sprayed on 130.0 g of magnesium aluminosilicate in a fluid bed Strea-1. The granular product was sieved through sieve 0.7 mm. 400 g of the granular product was mixed with 271.2 g microcrystalline cellulose and in a turbula mixer for 3 minutes and subsequently mixed with 6.8 g of magnesium stearate for 0.5 min. The granulate was directly compressed into 11 mm tablets on a Diaf TM20. The tablets had a mean weight of 455 mg and strength 10 mg.
Mean tablet hardness: 62N. Disintegration time: 2.1 min.

### Examples 8-9 (not according to the invention)

### Tablets comprising a dispersion of lercanidipine

The following compositions were prepared:

| **Substance** | **Ingredient** | **Form. G mg** | **Form. H mg** |
|---|---|---|---|
| **Drug** | Lercanidipine HCl | 200 | 20.0 |
| **Vehicle 1** | Gelucire 44/14 | 180.0 | 180.0 |
| **Carrier** | Magnesium aluminosilicate | 111.0 | 106.3 |
| | Mg stearate | 4.5 | 10.6 |
| **Excipients** | Cellulose microcryst. (Avicel PH102) | 135.2 | 105.6 |
| | Hypromellose (Metolose 90SH100) | - | 68.6 |
| | Hypromellose (Metolose90SH15000) | - | 37.0 |
| **Total** | | 450.7 | 528.1 |
| **Hardness** | N | 48 | 52 |
| **Disintegration time** | Minutes | 19 | - |
| **Diameter** | Mm | 12 | oblong |

### Formulation G:

23 g of lercanidipine was suspended in 207 g of Gelucire 44/14 at 60°C in a Turbula mixer. The solid solution was sprayed on 90 g of magnesium aluminosilicate in a fluid bed Strea-1. The granular product was sieved through sieve 0.7 mm. 100 g of the granular product was mixed with 43.5 g microcrystalline cellulose and in a turbula mixer for 3 minutes and subsequently mixed with 1.5 g of magnesium stearate for 0.5 min. The granulate was directly compressed into 12 mm tablets on a Diaf TM20. The tablets had a mean weight of 459 mg and strength 20 mg.
Mean tablet hardness: 48N. Disintegration time: 19 min.

### Formulation H:

26.1 g of lercanidipine was suspended in 235.3 g of Gelucire 44/14 at 60°C in a Turbula mixer. The solid solution was homogenized for 3 min in an Ultra-Turrax and subsequently sprayed on 130 g of magnesium aluminosilicate in a fluid bed Strea-1. The granular product was sieved through sieve 0.7 mm. 120 g of the granular product was mixed with 41.4 g of microcrystalline cellulose, 26.9 g of Metolose 100cP and 14.5 g of Metolose 15000cP in a turbula mixer for 3 minutes and subsequently mixed with 4.1 g of magnesium stearate for 0.5 min. The granulate was directly compressed into oblong 8 x 18 mm tablets (compound cup) on a Diaf TM20. The tablets had a mean weight of 527 mg and strength 20 mg.
Mean tablet hardness: 52N.

### Example 10

### Controlled release tablets

Tablets (uncoated) of 20mg strength and having the following controlled release (CR) formulations were prepared as described in Examples 4-5 and 8-9, respectively (compositions J, K, L, M):

| **Substance** | **Ingredient** | **J mg** CR_{fast} | **K mg** CR_{fast} | **L mg** CR_{slow} | **M mg** CR_{slow} |
|---|---|---|---|---|---|
| **Drug** | Lercanidipine HCl | 20.0 | 20.0 | 20.0 | 20.0 |
| **Vehicle** | Gelucire® 44/14 | 180.0 | - | 180.0 | - |
| | Glycerol monolaurate | - | 230.0 | - | 230.0 |
| **Carrier** | Magnesium aluminometasilicate | 107.7 | 134.6 | 107.7 | 134.6 |
| **Excipients** | Mg stearate | 5.2 | 8.7 | 10.6 | 9.9 |
| | Cellulose microcryst. (Avicel PH102) | 104.3 | - | 106.1 | - |
| | Metolose 90SH100 (hypromellose) | 104.3 | 43.7 | 69.0 | 98.6 |
| | Metolose90SH15000 (hypromellose) | - | - | 37.1 | - |
| **Total** | | 521.5 | 437.0 | 530.5 | 493.1 |
| **Hardness** | N | 59 | 65 | 50 | 75 |
| **Diameter** | Mm | oval | 8 x 18.8 | 8 x 18 | oval |

### Example 11

### Dissolution tests

The inventive controlled release tablet formulations J, K, L, M of example 10 were subjected to the USP II dissolution test (paddle method) using 100 rpm and as medium: 0.3% polysorbate 80 in 0.1N HCl

| % Dissolved (hours) | **J** | **K** | **L** | **M** |
|---|---|---|---|---|
| 20% | 2.5 | 2.0 | 4.5 | 2.0 |
| 40% | 4.0 | 3.0 | 6.7 | 3.1 |
| 80% | 5.0 | 8.5 | 8.5 | 8.0 |

### Example 12

### Stability tests

Samples of the inventive tablet formulations K and M of example 10 were stored under the following conditions, respectively, and subjected to a dissolution (stability) test as described in Methods after 1 month and 3 months of storage. All formulations fulfill the criteria below (% dissolved is the percentage of lercanidipine dissolved after 4 hours):

| Months | % dissolved | |
|---|---|---|
| | 25°C and 60% RH | 30°C and 65% RH |
| 0 | K: 50 ± 10 | K: 50 ± 10 |
| | M: 55 ± 10 | M: 55 ± 10 |
| 1 | K: 50 ± 10 | K: 50 ± 10 |
| | M: 55 ± 10 | M: 55 ± 10 |
| 3 | K: 50 ± 10 | K: 50 ± 10 |
| | M: 55 ± 10 | M: 55 ± 10 |

Samples of the inventive 20 mg tablet formulations J, K, L, M of example 10 were stored under the following conditions, respectively, and subjected to lercanidipine assay with the following results:

| Months | mg lercanidipine HCl | |
|---|---|---|
| | 25°C and 60% RH | 30°C and 65% RH |
| 0 | 19.0-21.0 | 19.0-21.0 |
| 1 | 18.0-21.0 | 18.0-21.0 |
| 3 | 18.0-21.0 | 18.0-21.0 |

Samples of the inventive tablet formulations J, K, L, M of example 10 were stored under the following conditions, respectively, and subjected to a degradation product test according to Ph. Eur. (Degradation products 1, B, 3 and Unknown accumulated into Total Degradation Product; HPLC method) with the following results:

| Months | Total Degradation Product, %w/w, impurity | |
|---|---|---|
| | 25°C and 60% RH | 30°C and 65% RH |
| 0 | ≤ 3.0 | ≤ 3.0 |
| 1 | ≤ 3.0 | ≤ 3.0 |
| 3 | ≤ 3.0 | ≤ 3.0 |

### Example 13

### In vivo bioavailability in dogs, Formulations B (according to the invention) and G (not according to the invention)

The comparison formulation is Zanidip® as disclosed in US-A1-2003/0180355, Table 3:

| **Ingredient** | **Zanidip mg** |
|---|---|
| Lercanidipine HCl | 10.0 |
| Lactose monohydrate | 30.0 |
| Sodium starch glycolate | 15.5 |
| Mg stearate | 1.0 |
| Cellulose microcryst. (Avicel PH102) | 39.0 |
| PVP (Povidone K 30) | 4.5 |
| Total | 100.0 |

An in vivo study of formulation B of example 3 and formulation G of example 8-9, 20 mg in Beagle dogs, performed as described above under Methods, relative to Zanidip®, gave the following results:
Mean blood concentrations (ng/mL, average of four dogs) of Lercanidipine after administration of the dosage forms:

| **Time** | **Formulation** | |
|---|---|---|
| **(hr)** | **Zanidip (20mg)** | **G (20 mg)** |
| | **Conc. (ng/ml)** | **Conc. (ng/ml)** |
| 0 | 0 | 0 |
| 0.25 | 0.743 ± 1.245 | 0.305 ± 0.610 |
| 0.5 | 4.323 ± 5.412 | 12.160 ± 14.736 |
| 1.0 | 18.688 ± 17.587 | 32.000 ± 12.184 |
| 2.0 | 15.443 ± 13.208 | 13.945 ± 5.709 |
| 3.0 | 8.065 ± 6.827 | 7.420 ± 2.969 |
| 4.0 | 4.363 ± 3.597 | 4.628 ± 1.566 |
| 6.0 | 2.029 ± 1.504 | 1.873 ± 0.696 |
| 8.0 | 1.033 ± 0.748 | 1.017 ± 0.338 |
| 24.0 | 0.176 ± 0.204 | 0.115 ± 0. 134 |

### Formulation G:

Relative bioavailability based on AUC (formulation G/Zanidip®) : 270%
Relative cₘₐₓ based on AUC (formulation G/Zanidip®) : 383%

### Formulation B:

Relative bioavailability based on AUC (formulation B/Zanidip@) : 142%
Relative cₘₐₓ based on AUC (formulation B/Zanidip®) : 149%

### Example 14

### In vivo bioavailability in dogs, Formulations C, E and F

An in vivo study of formulation C of example 4-5 and formulations E and F of example 6-7,20 mg (2 x 10 mg) in Beagle dogs, performed as described above under Methods, relative to Zanidip® formulation as disclosed in example 14, gave the following results.

Formulation C: Mean blood concentrations (ng/mL, average of four dogs) of Lercanidipine after administration of the dosage forms:

| **Time** | **Formulation** | |
|---|---|---|
| **(hr)** | **Zanidip (20mg)** | **C (20 mg)** |
| | **Conc. (ng/ml)** | **Conc. (ng/ml)** |
| 0 | 0 | 0 |
| 0.25 | 0.760 ± 0.934 | 1.622 ± 1.636 |
| 0.5 | 4.428 ± 3.559 | 15.0 ± 10.988 |
| 1.0 | 24.910 ± 16.758 | 26.25 ± 7.59 |
| 2.0 | 12.778 ± 10.651 | 14.473 ± 3.848 |
| 3.0 | 5.573 ± 4.686 | 4.99 ± 1.898 |
| 4.0 | 2.720 ± 2.073 | 3.008 ± 0.767 |
| 6.0 | 1.076 ± 0.631 | 1.173 ± 0.216 |
| 8.0 | 0.650 ± 0.350 | 0.718 ± 0.153 |
| 12.0 | 0.307 ± 0.305 | 0.518 ± 0.140 |
| 24.0 | 0.100 ± 0.199 | 0.143 ± 0.171 |

### Formulation C:

Relative bioavailability based on AUC (formulation C/Zanidip®): 163%
Relative cₘₐₓ based on AUC (formulation C/Zanidip®): 190%

### Formulation E:

Relative bioavailability based on AUC (formulation E/Zanidip®): 138%
Relative cₘₐₓ based on AUC (formulation E/Zanidip®): 135%

Formulation F: Mean blood concentrations (ng/mL, average of four dogs) of Lercanidipine after administration of the dosage forms:

| **Time** | **Formulation** | |
|---|---|---|
| **(hr)** | **Zanidip (20mg)** | **F (20 mg)** |
| | **Conc. (ng/ml)** | **Conc. (ng/ml)** |
| 0 | 0 | 0 |
| 0.25 | 0.760 ± 0.934 | 2.119 ± 3.251 |
| 0.5 | 4.428 ± 3.559 | 8.18 ± 11.808 |
| 1.0 | 24.910 ± 16.758 | 18.338 ± 16.226 |
| 2.0 | 12.778 ± 10.651 | 18.625 ± 9.665 |
| 3.0 | 5.573 ± 4.686 | 8.198 ± 5.435 |
| 4.0 | 2.720 ± 2.073 | 6.350 ± 6.739 |
| 6.0 | 1.076 ± 0.631 | 1.994 ± 1.273 |
| 8.0 | 0.650 ± 0.350 | 0.979 ± 0.640 |
| 12.0 | 0.307 ± 0.305 | 0.607 ± 0.490 |
| 24.0 | 0.100 ± 0.199 | 0.136 ± 0.271 |

### Formulation F:

Relative bioavailability based on AUC (formulation F/Zanidip®) : 140% Relative cₘₐₓ based on AUC (formulation F/Zanidip®) : 125%.

## Claims

1. A pharmaceutical composition comprising lercanidipine or a pharmaceutically acceptable salt thereof as an active substance and a pharmaceutically acceptable vehicle selected among glyceryl monolaurate, glyceryl monocaprylate and glyceryl (mono)caprate, wherein the active ingredient is:
fully dissolved in the vehicle to form a solid solution at ambient temperature.

2. A pharmaceutical composition according to claim 1 in the form of particles.

3. A pharmaceutical composition according to claim 1, wherein the concentration of active substance in the vehicle is less than 30 w/w%, based on the total weight of the active substance and the vehicle.

4. A pharmaceutical composition according to claim 1, wherein the concentration of active substance in the vehicle is at least 1 w/w%, based on the total weight of the active substance and the vehicle.

5. A controlled release pharmaceutical composition according to any of claims 1 to 4.

6. A solid dosage form comprising the pharmaceutical composition according to claim 1 and one or more pharmaceutically acceptable excipients.

7. A solid dosage form according to claim 6, which provides an AUC value relative to that of commercially available Zanidip® tablets of at least 1.1, or at least 1.2, or at least 1.3, or at least 1.4, or at least 1.5, or at least 1.75 or more, or at least 2.0, or at least 2.5, or at least 3.0, the AUC values being determined under similar conditions.

8. A solid dosage form according to claim 6, which provides a cₘₐₓ value relative to that of commercially available Zanidip® tablets of at least 1.1, or at least 1.2, or at least 1.3, or at least 1.4, or at least 1.5, or at least 1.6 or more, or at least 2.0, or at least 2.5, or at least 3.0, the cₘₐₓ values being determined under similar conditions.

9. A solid dosage form according to claim 6 in the form of tablets, beads, capsules, grains, pills, granulates, granules, powder, pellets, sachets or troches.

10. A solid dosage form according to claim 6, which is which is a unit dosage form for oral, buccal or sublingual administration.

11. A solid dosage form according to claim 6, wherein the pharmaceutically acceptable excipient is selected from the group consisting of fillers, disintegrants, binders, diluents, lubricants and glidants.

12. A solid dosage form according to claim 6, which further comprises a pharmaceutically acceptable additive selected from the group consisting of flavoring agents, coloring agents, taste-masking agents, pH-adjusting agents, buffering agents, preservatives, stabilizing agents, anti-oxidants, wetting agents, humidity-adjusting agents, surface-active agents, suspending agents, absorption enhancing agents.

13. A solid dosage form according to claim 6 wherein the one or more pharmaceutically acceptable excipients are selected from the group consisting of silica acid and derivatives or salts thereof including silicates, silicon dioxide and polymers thereof; magnesium aluminosilicate and magnesium aluminometasilicate, bentonite, kaolin, magnesium trisilicate, montmorillonite and saponite.

14. A solid dosage form according to claim 6, wherein the one or more pharmaceutically acceptable excipients comprise an oily material.

15. A solid dosage form according to claim 14, wherein the concentration of the oily material in the dosage form is 5% w/w or more such as, e.g., 10% w/w or more, 15% w/w or more, 20% w/w or more, 25% w/w or more, 30% w/w or more, 35% w/w or more, 40% w/w or more, 45% w/w or more, 50 w/w or more, 55% w/w or more, 60% w/w or more, 65% w/w or more, 70% w/w or more, 75% w/w or more, 80% w/w or more, 85% w/w or more, 90% w/w or more or 95% w/w or more.

16. A solid dosage form according to claim 6, which upon oral administration to a mammal in need thereof releases the active substance in a controlled manner.

17. A solid dosage form according to claim 16, which does not exhibit a significant adverse food effect as evidenced by a value of (AUC_{fed}/AUC_{fasted}) of at least 0.85 with a lower 90% confidence limit of at least 0.75.

18. A solid dosage form according to claim 17, wherein the value of (AUC_{fed}/AUC_{fasted}) is at the most 3, such as, e.g. at the most 2.5, at the most 2.0, at the most 1.5, at the most 1, such as, e.g., 0.9 or more, 0.95 or more, 0.97 or more or 1 or more.

19. A solid dosage form according to claim 6, which upon oral administration to a mammal in need thereof releases the active substance in a controlled manner and reduces inter- and/or intra-individual variations compared to those of Zanidip® administered under the same conditions and in a dose that provides an equivalent therapeutic effect.

20. A solid dosage form according to claim 6, which releases at least 20% w/w of the total amount of the active substance within 8 hours, within 6 hours, within 4 hours, within 3 hours or within 2 hours, when tested in vitro according to the USP II dissolution test (paddle) using 0.3% polysorbate 80 in 0.1 M HCI as medium, 100 rpm.

21. A solid dosage form according to claim 6, which releases at least 40% w/w of the total amount of the active substance within 10 hours, within 8 hours, within 7 hours, within 6 hours, within 4 hours or within 3 hours, when tested in vitro according to the USP II dissolution test (paddle) using 0.3% polysorbate 80 in 0.1 M HCI as medium, 100 rpm.

22. A solid dosage form according to claim 6, which releases at least 55% w/w, 60% w/w or more, 65% w/w or more, 70% w/w or more, 75% w/w or more or 80% w/w or more of the total amount of the active substance within 24 hours, within 16 hours, within 12 hours, within 10 hours, within 9 hours, within 8 hours, or within 6 hours, when tested in vitro according to the USP II dissolution test (paddle) using 0.3% polysorbate 80 in 0.1 M HCI as medium, 100 rpm.

23. A solid dosage form according to claim 6, which upon oral administration to a mammal in need thereof in a controlled manner releases at least 20% w/w of the total amount of the active substance within 8 hours, within 6 hours, within 4 hours, within 3 hours or within 2 hours.

24. A solid dosage form according to claim 6, which the composition upon oral administration to a mammal in need thereof releases at least 40% w/w of the total amount of the active substance within 16 hours such as, e.g., within 12 hours, within 10 hours, within 8 hours, within 7 hours, within 6 hours, within 4 hours or within 3 hours.

25. A solid dosage form according to claim 6 which upon oral administration to a mammal in need thereof releases at least 55% w/w such as, e.g., about 60% w/w or more, 65% w/w or more, about 70% w/w or more, 75% w/w or more or 80% w/w or more of the total amount of the active substance within 24 hours such as, e.g., within 16 hours, within 12 hours, within 10 hours, within 9 hours, within 8 hours, or within 6 hours.

26. A solid dosage form according to claim 6, wherein the concentration of the pharmaceutical composition is in a range of from 5% to 100% w/w such as, e.g., from 10% to 90% w/w, from 15% to 85% w/w, from 20% to 80% w/w, from 25% to 80% w/w, from 30% to 80% w/w, from 35% to 80% w/w, from 40% to 75% w/w, from 45% to 75% w/w or from 50% to 70% w/w of the dosage form.

27. A solid dosage form according to claim 26, wherein the concentration of the pharmaceutical composition in particulate form is 50% w/w or more of the dosage form.

28. A solid dosage form according to claim 6, wherein the solid dosage form upon oral administration to a mammal in need thereof releases lercanidipine in a controlled manner and the solid dosage form being essentially bioequivalent with Zanidip® or a similar commercially available lercanidipine-containing product.

29. A solid dosage form according to claim 28, wherein the dosage form is administered in a dose that is at the most 85% w/w of the dose of lercanidipine administered in the form of Zanidip® or a similar commercially available lercanidipine containing product.

30. A method of manufacturing the solid oral dosage form of claim 6 comprising the steps of:
i) Bringing the vehicle in liquid form to obtain a liquid vehicle,
ii) Maintaining the liquid vehicle at a temperature below the melting point of the active substance,
iii) Dissolving the desired amount of active substance in the vehicle of i),
iv) Spraying the resulting solution onto a solid carrier having a temperature below the melting point of the vehicle to obtain a composition,
v) Mechanically working the resulting composition to obtain particles, i.e. a particulate material, and
vi) Optionally subjecting the particulate material to conventional methods for preparing solid dosage forms.

31. A composition according to claim 1 with enhanced oral bioavailability of lercanidipine or pharmaceutically acceptable salt thereof for the treatment of hypertension or angina pectoris.

32. Use of the composition according to claim 1 for the preparation of a delayed release oral solid dosage form, preferably tablets or capsules.

## Patentansprüche

1. Pharmazeutische Zusammensetzung mit einem Lercanidipin oder einem pharmazeutisch geeigneten Salz hiervon als aktive Substanz und einem pharmazeutisch geeigneten Trägerstoff, der ausgewählt ist aus Glyzeryl-Monolaurat, Glyceryl-Monocaprylat and Glyceryl-(mono)caprate, wobei der Wirkstoff
vollständig in dem Trägerstoff gelöst ist, um eine bei Umgebungstemperatur feste Lösung zu bilden.

2. Pharmazeutische Zusammensetzung nach Anspruch 1, in Form von Teilchen.

3. Pharmazeutische Zusammensetzung nach Anspruch 1, bei welcher die Konzentration des Wirkstoffs in dem Trägerstoff geringer ist als 30 w/w%, auf der Basis des Gesamtgewichts des Wirkstoffs und des Trägerstoffs.

4. Pharmazeutische Zusammensetzung nach Anspruch 1, bei welcher die Konzentration des Wirkstoffs in dem Trägerstoff wenigstens 1 w/w% ist, auf der Basis des Gesamtgewichts des Wirkstoffs und des Trägerstoffs.

5. Pharmazeutische Zusammensetzung mit kontrollierter Freigabe nach einem der Ansprüche 1 bis 4.

6. Feste Darreichungsform mit einer pharmazeutischen Zusammensetzung nach Anspruch 1 und einem oder mehreren pharmazeutisch geeigneten Hilfsstoffen.

7. Feste Darreichungsform nach Anspruch 6, welche einen AUC-Wert relativ zu dem von im Handel erhältlichen Zanidip®-Tabletten hat, mit wenigstens 1,1 oder wenigstens 1,2 oder wenigstens 1,3 oder wenigstens 1,4 oder wenigstens 1,5 oder wenigstens 1,75 oder mehr oder wenigstens 2,0 oder wenigstens 2,5 oder wenigstens 3,0, wobei die AUC-Werte unter ähnlichen Bedingungen bestimmt werden.

8. Feste Darreichungsform nach Anspruch 6, welche einen cₘₐₓ - Wert relativ zu dem von im Handel erhältlichen Zanidip®-Tabletten hat, mit wenigstens 1,1 oder wenigstens 1,2 oder wenigstens 1,3 oder wenigstens 1,4 oder wenigstens 1,5 oder wenigstens 1,6 oder mehr oder wenigstens 2,0 oder wenigstens 2,5 oder wenigstens 3,0 wobei die cₘₐₓ - Werte unter ähnlichen Bedingungen bestimmt werden.

9. Feste Darreichungsform nach Anspruch 6, in Form von Tabletten, Kügelchen, Kapseln, Körnern, Pillen, Granulaten, Körnchen, Pulver, Pellets, Beuteln oder Pastillen.

10. Feste Darreichungsform nach Anspruch 6, welche eine Dosierungseinheit für orale, bukkale oder sublinguale Verabreichung ist.

11. Feste Darreichungsform nach Anspruch 6, wobei der pharmazeutisch geeignete Hilfsstoff aus der Gruppe ausgewählt ist, die aus Füllstoffen, Desintegrationsmitteln, Bindern, Lösungsmittels, Schmiermitteln und Gleitmitteln besteht.

12. Feste Darreichungsform nach Anspruch 6, welche pharmazeutisch geeignete Additive aufweist, die aus der Gruppe ausgewählt sind, die aus Aromastoffen, Farbstoffen, Geschmack verbergenden Wirkstoffen, pH-einstellenden Wirkstoffen, Pufferstoffen, Konservierungsmitteln, Stabilisatoren, Antioxidantien, Befeuchtungsmitteln, Feuchtigkeit einstellenden Wirkstoffen, oberflächenaktiven Wirkstoffen, Suspensionsmitteln, absorptionsverstärkenden Wirkstoffen besteht.

13. Feste Darreichungsform nach Anspruch 6, wobei der eine oder mehrere pharmazeutisch geeignete Hilfsstoff/e aus der Gruppe ausgewählt ist/sind, die aus Kieselsäure und Derivaten oder Salzen hiervon, einschließlich Silikaten, Silikondioxide und Polymeren hiervon, Magnesiumaluminiumsilikat und Magnesiumaluminiummetasilikat, Bentonit, Kaolin, Magnesiumtrisilikat, Montmorillonit und Saponit besteht.

14. Feste Darreichungsform nach Anspruch 6, wobei der eine oder mehrere pharmazeutisch geeignete Hilfsstoff/e öliges Material enthält/enthalten.

15. Feste Darreichungsform nach Anspruch 14, wobei die Konzentration des öligen Materials in der Darreichungsform 5% w/w oder mehr, wie z. B. 10% w/w oder mehr, 15% w/w oder mehr, 20% w/w oder mehr, 25% w/w oder mehr, 30% w/w oder mehr, 35% w/w oder mehr, 40% w/w oder mehr, 45% w/w oder mehr, 50% w/w oder mehr, 55% w/w oder mehr, 60% w/w oder mehr, 65% w/w oder mehr, 70% w/w oder mehr, 75% w/w oder mehr, 80% w/w oder mehr, 85% w/w oder mehr, 90% w/w oder mehr, oder 95% w/w oder mehr beträgt.

16. Feste Darreichungsform nach Anspruch 6, welche bei oraler Darreichung an einen diese benötigenden Säuger die aktive Substanz in kontrollierter Art und Weise abgibt.

17. Feste Darreichungsform nach Anspruch 16, welche keinen signifikanten nachteiligen Nahrungsmitteleffekt zeigt, wie er durch einen Wert von (AUC_{fed}/AUC_{fasted}) von wenigstens 0,85 mit einer unter 90% liegenden Konfidenzgrenze von wenigstens 0,75 belegt ist.

18. Feste Darreichungsform nach Anspruch 17, wobei der Wert von (AUC_{fed}/AUC_{fasted}) höchstens 3, wie z. B. höchstens 2,5, höchstens 2,0, höchstens 1,5, höchstens 1, wie z. B. 0,9 oder mehr, 0,95 oder mehr, 0,97 oder mehr, oder 1 oder mehr beträgt.

19. Feste Darreichungsform nach Anspruch 6, welche bei oraler Darreichung an einen diese benötigenden Säuger die aktive Substanz in kontrollierter Art und Weise abgibt und inter- und/oder intra-individuelle Varianten reduziert, verglichen mit denen von Zanidip®, welches unter den gleichen Bedingungen und in einer Dosis gegeben wird, welche einen äquivalenten therapeutischen Effekt bewirkt.

20. Feste Darreichungsform nach Anspruch 6, welche wenigstens 20% w/w der Gesamtmenge der aktiven Substanz innerhalb von 8 Stunden, innerhalb von 6 Stunden, innerhalb von 4 Stunden, innerhalb von 3 Stunden oder innerhalb von 2 Stunden abgibt, gemessen in vitro gemäß dem USP II Dissolution Test (paddle) unter Verwendung von 0,3% Polysorbat 80 in 0,1 M HCl als Medium, bei 100 rpm.

21. Feste Darreichungsform nach Anspruch 6, welche wenigstens 40% w/w der Gesamtmenge der aktiven Substanz innerhalb von 10 Stunden, innerhalb von 8 Stunden, innerhalb von 7 Stunden, innerhalb von 6 Stunden, innerhalb von 4 Stunden oder innerhalb von 3 Stunden abgibt, gemessen in vitro gemäß dem USP II Dissolution Test (paddle) unter Verwendung von 0,3% Polysorbat 80 in 0,1 M HCl als Medium, bei 100 rpm.

22. Feste Darreichungsform nach Anspruch 6, welche wenigstens 55% w/w, 60% w/w oder mehr, 65% w/w oder mehr, 70% w/w oder mehr, 75% w/w oder mehr oder 80% w/w oder mehr der Gesamtmenge der aktiven Substanz innerhalb von 24 Stunden, innerhalb von 16 Stunden, innerhalb von 12 Stunden, innerhalb von 10 Stunden, innerhalb von 9 Stunden, innerhalb von 8 Stunden oder innerhalb von 6 Stunden abgibt, gemessen in vitro gemäß dem USP II Dissolution Test (paddle) unter Verwendung von 0,3% Polysorbat 80 in 0,1 M HCl als Medium, bei 100 rpm.

23. Feste Darreichungsform nach Anspruch 6, welche bei oraler Darreichung an einen diese benötigenden Säuger wenigstens 20% w/w der Gesamtmenge der aktiven Substanz innerhalb von 8 Stunden, innerhalb von 6 Stunden, innerhalb von 4 Stunden, innerhalb von 3 Stunden oder innerhalb von 2 Stunden in kontrollierter Art und Weise abgibt.

24. Feste Darreichungsform nach Anspruch 6, wobei die Zusammensetzung bei oraler Darreichung an einen diese benötigenden Säuger wenigstens 40% w/w der Gesamtmenge der aktiven Substanz innerhalb von 16 Stunden, wie z. B. innerhalb von 12 Stunden, innerhalb von 10 Stunden, innerhalb von 8 Stunden, innerhalb von 7 Stunden, innerhalb von 6 Stunden, innerhalb von 4 Stunden oder innerhalb von 3 Stunden in kontrollierter Art und Weise abgibt.

25. Feste Darreichungsform nach Anspruch 6, welche bei oraler Darreichung an einen diese benötigenden Säuger wenigstens 55% w/w, wie z. B. 60% w/w oder mehr, 65% w/w oder mehr, 70% w/w oder mehr, 75% w/w oder mehr oder 80% w/w oder mehr der Gesamtmenge der aktiven Substanz innerhalb von 24 Stunden, wie z. B. innerhalb von 16 Stunden, innerhalb von 12 Stunden, innerhalb von 10 Stunden, innerhalb von 9 Stunden, innerhalb von 8 Stunden oder innerhalb von 6 Stunden abgibt.

26. Feste Darreichungsform nach Anspruch 6, wobei die Konzentration der pharmazeutischen Zusammensetzung im Bereich von 5% bis 100% w/w, wie z. B. 10% bis 90% w/w, 15% bis 85% w/w, 20% bis 80% w/w, 25% bis 80% w/w, 30% bis 80% w/w, 35% bis 80% w/w, 40% bis 75% w/w, 45% bis 75% w/w oder 50% bis 70% w/w der Darreichungsform liegt.

27. Feste Darreichungsform nach Anspruch 26, wobei die Konzentration der pharmazeutischen Zusammensetzung in Teilchenform bei 50% oder mehr der Darreichungsform beträgt.

28. Feste Darreichungsform nach Anspruch 6, wobei die feste Darreichungsform bei oraler Darreichung an einen diese benötigenden Säuger Lercanidipin in kontrollierter Art und Weise abgibt und die feste Darreichungsform im Wesentlichen bioäquivalent mit Zanidip® oder einem ähnlichen im Handel erhältlichen Lercanidipin enthaltenden Produkt ist.

29. Feste Darreichungsform nach Anspruch 28, wobei die Darreichungsform in einer Dosis verabreicht wird, welche höchstens 85% w/w der Dosis von Lercanidipin beträgt, welches in Form von Zanidip® oder einem ähnlichen im Handel erhältlichen Lercanidipin enthaltenden Produkt beträgt.

30. Verfahren zum Herstellen der festen Darreichungsform nach Anspruch 6, mit folgenden Schritten:
i) Bringen der Trägerstoffs in flüssige Form, um einen flüssigen Trägerstoff zu erhalten,
ii) Halten des flüssigen Trägerstoffs bei einer Temperatur unterhalb des Schmelzpunktes der aktiven Substanz,
iii) Lösen der gewünschten Menge an aktiver Substanz in dem Trägerstoff von i),
iv) Sprühen der sich ergebenden Lösung auf einen festen Träger mit einer Temperatur unterhalb des Schmelzpunktes des Trägerstoffs, um eine Verbindung zu erhalten,
v) Mechanisches Bearbeiten der sich ergebenden Lösung, um Teilchen, d. h. teilchenartiges Material zu erhalten,
und
vi) Optionales Unterwerfen des teilchenartigen Material konventionellen Verfahren zum Herrichten von feste Darreichungsformen.

31. Zusammensetzung nach Anspruch 1 mit verbesserter oraler Bioeignung von Lercanidipin oder pharmazeutisch geeignetem Salz hiervon für die Behandlung von Bluthochdruck oder Angina pectoris.

32. Verwendung der Zusammensetzung nach Anspruch 1 für die Herstellung einer oralen festen Darreichungsform, vorzugsweise Tabletten oder Kapseln, mit verzögerter Wirkstoffabgabe.

## Revendications

1. Composition pharmaceutique comprenant de la lercanidipine ou un sel de celle-ci pharmaceutiquement acceptable en tant que substance active et un véhicule pharmaceutiquement acceptable sélectionné parmi le monolaurate de glycéryle, le monocaprylate de glycéryle et le (mono)caprate de glycéryle, dans laquelle l'ingrédient actif est :
complètement dissous dans le véhicule afin de former une solution solide à température ambiante.

2. Composition pharmaceutique selon la revendication 1 sous la forme de particules.

3. Composition pharmaceutique selon la revendication 1, dans laquelle la concentration de la substance active dans le véhicule est inférieure à 30 % p/p, en se basant sur le poids total de la substance active et du véhicule.

4. Composition pharmaceutique selon la revendication 1, dans laquelle la concentration de la substance active dans le véhicule est d'au moins 1 % p/p, en se basant sur le poids total de la substance active et du véhicule.

5. Composition pharmaceutique à libération contrôlée selon l'une quelconque des revendications 1 à 4.

6. Forme posologique solide comprenant la composition pharmaceutique selon la revendication 1 et un ou plusieurs excipients pharmaceutiquement acceptables.

7. Forme posologique solide selon la revendication 6, qui permet d'obtenir une valeur ASC, par rapport à celle de comprimés de Zanidip® disponibles dans le commerce, d'au moins 1,1, ou au moins 1,2, ou au moins 1,3, ou au moins 1,4, ou au moins 1,5, ou au moins 1,75 ou plus, ou au moins 2,0, ou au moins 2,5, ou au moins 3,0, les valeurs ASC étant déterminées dans des conditions similaires.

8. Forme posologique solide selon la revendication 6, qui permet d'obtenir une valeur cₘₐₓ, par rapport à celle de comprimés de Zanidip® disponibles dans le commerce, d'au moins 1,1, ou au moins 1,2, ou au moins 1,3, ou au moins 1,4, ou au moins 1,5, ou au moins 1,6 ou plus, ou au moins 2,0, ou au moins 2,5, ou au moins 3,0, les valeurs cₘₐₓ étant déterminées dans des conditions similaires.

9. Forme posologique solide selon la revendication 6, sous la forme de comprimés, billes, capsules, granulés, pilules, granulats, granules, poudre, pellets, sachets ou pastilles.

10. Forme posologique solide selon la revendication 6, qui est une forme posologique unitaire pour une administration orale, buccale ou sublinguale.

11. Forme posologique solide selon la revendication 6, dans laquelle l'excipient pharmaceutiquement acceptable est sélectionné dans le groupe consistant en des charges, des délitants, des liants, des diluants, des lubrifiants et des agents glissants.

12. Forme posologique solide selon la revendication 6, qui comprend en outre un additif pharmaceutiquement acceptable sélectionné dans le groupe consistant en des agents aromatisants, des agents colorants, des agents de masquage du goût, des agents d'ajustement du pH, des agents tampon, des conservateurs, des agents stabilisateurs, des antioxydants, des agents mouillants, des agents d'ajustement de l'humidité, des agents tensioactifs, des agents de suspension, des agents améliorant l'absorption.

13. Forme posologique solide selon la revendication 6, dans laquelle le un ou plusieurs excipients pharmaceutiquement acceptables sont sélectionnés dans le groupe consistant en l'acide de silice et des dérivés ou sels de celui-ci comprenant des silicates, le dioxyde de silicium et des polymères de ceux-ci ; l'aluminosilicate de magnésium et l'aluminométasilicate de magnésium, la bentonite, le kaolin, le trisilicate de magnésium, la montmorillonite et la saponite.

14. Forme posologique solide selon la revendication 6, dans laquelle le un ou plusieurs excipients pharmaceutiquement acceptables comprennent un matériau huileux.

15. Forme posologique solide selon la revendication 14, dans laquelle la concentration du matériau huileux dans la forme posologique est de 5 % p/p ou plus tel que, par exemple, 10 % p/p ou plus, 15 % p/p ou plus, 20 % p/p ou plus, 25 % p/p ou plus, 30 % p/p ou plus, 35 % p/p ou plus, 40 % p/p ou plus, 45 % p/p ou plus, 50 % p/p ou plus, 55 % p/p ou plus, 60 % p/p ou plus, 65 % p/p ou plus, 70 % p/p ou plus, 75 % p/p ou plus, 80 % p/p ou plus, 85 % p/p ou plus, 90 % p/p ou plus ou 95 % p/p ou plus.

16. Forme posologique solide selon la revendication 6 qui, suite à une administration orale à un mammifère en ayant besoin, libère la substance active de manière contrôlée.

17. Forme posologique solide selon la revendication 16, qui n'exhibe pas d'effet indésirable significatif lié aux aliments tel que démontré par une valeur (ASC_{non à jeun}/ASC_{à jeun}) d'au moins 0,85 avec une limite inférieure de l'intervalle de confiance à 90 % d'au moins 0,75.

18. Forme posologique solide selon la revendication 17, où la valeur (ASC_{non à jeun}/ASC_{à jeun}) est de 3 au maximum, tel que, par exemple, 2,5 au maximum, 2,0 au maximum, 1,5 au maximum, 1 au maximum, tel que, par exemple, 0,9 ou plus, 0,95 ou plus, 0,97 ou plus ou 1 ou plus.

19. Forme posologique solide selon la revendication 6 qui, suite à une administration orale à un mammifère en ayant besoin, libère la substance active de manière contrôlée et réduit les variations inter- et/ou intra-individu(s) par comparaison à celles du Zanidip® administré dans les mêmes conditions et en une dose qui permet d'obtenir un effet thérapeutique équivalent.

20. Forme posologique solide selon la revendication 6, qui libère au moins 20 % p/p de la quantité totale de la substance active en 8 heures, en 6 heures, en 4 heures, en 3 heures ou en 2 heures, lorsqu'elle est testée *in vitro* conformément au test de dissolution USP II (palette) en utilisant du polysorbate 80 à 0,3 % dans du HCl 0,1 M en tant que milieu, à 100 tr/min.

21. Forme posologique solide selon la revendication 6, qui libère au moins 40 % p/p de la quantité totale de la substance active en 10 heures, en 8 heures, en 7 heures, en 6 heures, en 4 heures ou en 3 heures, lorsqu'elle est testée *in vitro* conformément au test de dissolution USP II (palette) en utilisant du polysorbate 80 à 0,3 % dans du HCl 0,1 M en tant que milieu, à 100 tr/min.

22. Forme posologique solide selon la revendication 6, qui libère au moins 55 % p/p, 60 % p/p ou plus, 65 % p/p ou plus, 70 % p/p ou plus, 75 % p/p ou plus ou 80 % p/p ou plus de la quantité totale de la substance active en 24 heures, en 16 heures, en 12 heures, en 10 heures, en 9 heures, en 8 heures, ou en 6 heures, lorsqu'elle est testée *in vitro* conformément au test de dissolution USP II (palette) en utilisant du polysorbate 80 à 0,3 % dans du HCl 0,1 M en tant que milieu, à 100 tr/min.

23. Forme posologique solide selon la revendication 6 qui, suite à une administration orale à un mammifère en ayant besoin, libère de manière contrôlée au moins 20 % p/p de la quantité totale de la substance active en 8 heures, en 6 heures, en 4 heures, en 3 heures ou en 2 heures.

24. Forme posologique solide selon la revendication 6, où la composition, suite à une administration orale à un mammifère en ayant besoin, libère au moins 40 % p/p de la quantité totale de la substance active en 16 heures tel que, par exemple, en 12 heures, en 10 heures, en 8 heures, en 7 heures, en 6 heures, en 4 heures ou en 3 heures.

25. Forme posologique solide selon la revendication 6 qui, suite à une administration orale à un mammifère en ayant besoin, libère au moins 55 % p/p tel que, par exemple, environ 60 % p/p ou plus, 65 % p/p ou plus, environ 70 % p/p ou plus, 75 % p/p ou plus ou 80 % p/p ou plus de la quantité totale de la substance active en 24 heures tel que, par exemple, en 16 heures, en 12 heures, en 10 heures, en 9 heures, en 8 heures, ou en 6 heures.

26. Forme posologique solide selon la revendication 6, dans laquelle la concentration de la composition pharmaceutique est dans une plage comprise entre 5 % et 100 % p/p tel que, par exemple, entre 10 % et 90 % p/p, entre 15 % et 85 % p/p, entre 20 % et 80 % p/p, entre 25 % et 80 % p/p, entre 30 % et 80 % p/p, entre 35 % et 80 % p/p, entre 40 % et 75 % p/p, entre 45 % et 75 % p/p ou entre 50 % et 70 % p/p de la forme posologique.

27. Forme posologique solide selon la revendication 26, dans laquelle la concentration de la composition pharmaceutique sous forme particulaire est 50 % p/p ou plus de la forme posologique.

28. Forme posologique solide selon la revendication 6, où la forme posologique solide, suite à une administration orale à un mammifère en ayant besoin, libère de la lercanidipine de manière contrôlée et la forme posologique solide est essentiellement bioéquivalente au Zanidip® ou à un produit contenant de la lercanidipine similaire disponible dans le commerce.

29. Forme posologique solide selon la revendication 28, où la forme posologique est administrée en une dose qui est au maximum 85 % p/p de la dose de lercanidipine administrée sous la forme de Zanidip® ou d'un produit contenant de la lercanidipine similaire disponible dans le commerce.

30. Procédé de fabrication de la forme posologique orale solide selon la revendication 6, comprenant les étapes consistant à :
i) mettre le véhicule sous forme liquide afin d'obtenir un véhicule liquide,
ii) maintenir le véhicule liquide à une température inférieure au point de fusion de la substance active,
iii) dissoudre la quantité souhaitée de substance active dans le véhicule de i),
iv) pulvériser la solution résultante sur un support solide possédant une température inférieure au point de fusion du véhicule afin d'obtenir une composition,
v) travailler mécaniquement la composition résultante afin d'obtenir des particules, c'est-à-dire un matériau particulaire, et
vi) facultativement soumettre le matériau particulaire à des procédés conventionnels de préparation de formes posologiques solides.

31. Composition selon la revendication 1 présentant une biodisponibilité orale améliorée de la lercanidipine ou d'un sel de celle-ci pharmaceutiquement acceptable pour le traitement de l'hypertension ou de l'angine de poitrine.

32. Utilisation de la composition selon la revendication 1 pour la préparation d'une forme posologique solide orale à libération retardée, de préférence des comprimés ou des capsules.
